# EUROPEAN PATENT APPLICATION

(11) **EP 3 231 799 A1**
(43) Date of publication of application: **18.10.2017**
(21) Application number: 16165301.9
(22) Date of filing: 14.04.2016
(51) Int. Cl.: C07D 401/14, C07D 413/14, C07D 403/14, A61K 31/506, A61P 35/00, A61P 29/00, A61P 31/00

(54) **4-(AZETIDIN-1-YL)PYRIMIDINE DERIVATIVES WITH ANTI-MITOTIC AND ANTI-PROLIFERATIVE ACTIVITY**

(71) Applicant: Universität Basel, 4001 Basel (CH)
(72) Inventor: Bohnacker, Thomas, 4056 Basel (CH); Rageot, Denise, 68300 Saint-Louis (FR); Sele, Alexander Markus, 4057 Basel (CH); Beaufils, Florent, 68870 Bartenheim (FR); Wymann, Matthias, 3012 Bern (CH)
(74) Representative: Latscha Schöllhorn Partner AG

(57) **Abstract**

The invention relates to 4-(azetidin-1-yl)pyrimidyl compounds of formula (I), 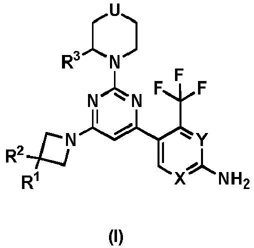 wherein X and Y are, independently of each other CH or N; U is O, CHOH, CHCH₂OH, C(CH₃)OH, CO, CHNH₂; R¹ and R² are, independently of each other H, F or CH₃; and R³ is H or CH₃; and related compounds with anti-mitotic and anti-proliferative properties, and methods of making use of such compounds in therapy or diagnostics to attenuate undesired cell proliferation, growth, migration, metastasis tumor formation, and inflammatory conditions. Additionally, methods are disclosed of treating diseases associated with undesired angiogenesis and undesired proliferation, and methods of treating infectious and inflammatory diseases using such compounds.

## Description

### Field of the Invention

The invention relates to novel 4-(azetidin-1-yl)pyrimidyl compounds, which exhibit anti-mitotic and anti-proliferative properties, and methods of making and using such compounds in therapy or diagnostics to attenuate undesired cell proliferation, growth, migration, metastasis tumor formation, and inflammatory conditions. Additionally, methods are disclosed of treating diseases associated with undesired angiogenesis and undesired proliferation, and methods of treating infectious and inflammatory disease.

### Background of the Invention

Anti-cancer therapy utilizes a combination of therapeutic interventions such as surgery, radiation therapy and chemotherapy. Surgery and radiation therapy are generally confined locally to the main site of tumor growth, while chemotherapy is applied to prevent tumor re-growth or against distant tumor foci. Chemotherapeutic agents are also used to reduce tumor growth to manage disease progression when radiotherapy or surgery is not an option.

Inhibitors of mitosis (also called mitotic inhibitors or anti-mitotics) are important therapeutics for the treatment of diseases, and they are used in treating cancer, as well as anti-gout, anti-fungus agents and treating restenosis. These inhibitors disrupt mitosis and block cell division. In cancer, inhibitors of mitosis can block tumor growth and lead to tumor cell apoptosis or exit from mitosis followed by cell death.

Many inhibitors of mitosis have been described. Some inhibitors of mitosis are tubulin-binding agents. Anti-tubulin agents act on tubulin, a protein that is necessary for mitosis. Anti-tubulin agents include vinca alkaloids, taxanes and epothilones. Non-tubulin targeted inhibitors of mitosis have also been investigated as cancer therapeutics. Different inhibitors of mitosis affect different portions of the cell cycle; and sometimes other functions outside of mitosis. For instance, anti-tubulin agents can affect non-mitotic cytoskeletal functions in proliferating cells and in terminally differentiated cells. Peripheral neurotoxicity has been associated with tubulin agents. Thus, different inhibitors of mitosis may have different toxicities.

Vinca alkaloids inhibit microtubule polymerization, which thereby inhibits mitosis. Vinca alkaloids include vinblastine, vincristine, vindesine and vinorelbine. Vinblastine has been used to treat certain kinds of cancer, including Hodgkin's lymphoma, non-small cell lung cancer, breast cancer and testicular cancer. Vincristine has been used to treat certain kinds of cancer, including lymphoma, breast cancer, lung cancer and acute lymphoblastic leukemia. Vinblastine and vincristine have also been used in palliative regimens for some or the major solid tumors. Vindesine has been used to treat certain kinds of cancer, including leukemia, lymphoma, melanoma, breast cancer and lung cancer.

Taxanes stabilize microtubules, thereby inactivating the microtubule function of a cell and inhibiting cell division. Taxanes include paclitaxel (including abraxane) and docetaxel. Paclitaxel is used to treat certain kinds of cancer, including lung cancer, ovarian cancer, breast cancer and advanced forms of Kaposi's sarcoma. Docetaxel is used to treat certain kinds of cancer, including breast cancer, ovarian cancer and non-small cell lung cancer.

Additionally, colchicine is an inhibitor of mitosis. Colchicine inhibits cell division by blocking microtubule polymerization. Colchicine is also used to treat gout.

Epothilones are a class of microtubule-stabilizing chemotherapeutic agents with activity in paclitaxel-resistant cancer cell lines. Epothilones include epothilone A, epothilone B, epothilone D and the epothilone analog ixabepilone.

Aurora kinases, including aurora A, aurora B and aurora C, are serine / threonine kinases that function in mitosis. Aurora kinases have been targeted as inhibitors of mitosis. Aurora A has its function in the pro-phase of mitosis and is required for the centrosomes to function correctly. Aurora B functions in the attachment of the mitotic spindle to the centromere. Aurora kinase inhibitors include CYC-116, AS-703569 (or R-763), MLN-8054, PHA-739358, AT-9283, SNS-314, AZD-1152-HQPA, 3MLN-8237, KW-2449, PF-3814735, ENMD-2076 (or ENMD-981693), PHA-739385.

For a review on mitotic inhibitors, see Chan KS, Koh CG, and Li HY, Mitosis-targeted anti-cancer therapies: where they stand, Cell Death Dis 3:e411(2012); Khrapunovich-Baine M et al., Hallmarks of molecular action of microtubule stabilizing agents: effects of epothilone B, ixabepilone, peloruside A, and laulimalide on microtubule conformation, J Biol Chem 286:11765-11778 (2011).

### Summary of the Invention

The invention relates to new 4-(azetidin-1-yl)pyrimidyl-based compounds and their use as therapeutic agents.

The invention also relates to mitosis inhibitor-induced mitotic arrest and other attenuated cell responses, including cell migration, adhesion and integrin activation, using such compounds.

The invention also relates to such microtubule-targeting compounds as chemotherapeutic agents with anti-cancer activity, pharmaceutical formulations thereof, which are potentially useful in treatment of disease, conditions and/or disorders modulated by augmented cell division, growth, migration, adhesion and metastasis. The compounds may inhibit tumor growth in mammals, and may be useful for treating human cancer patients.

Additionally to cancer treatment, the present invention also relates to the treatment of pathogenic cellular states caused by cell over-activation using such compounds. Molecules targeting microtubules can be used to treat a variety of hyper-proliferative diseases but also gout, autoimmune disease, arthritis, graft rejection, inflammatory bowel disease, cellular proliferation induced after a medical procedure, and other inflammatory and immune disorders.

The invention also relates to methods of using such compounds for *in vitro, in situ,* and *in vivo* diagnostic procedures or treatment of mammalian cells, organisms, or associated pathological conditions, or production processes.

The invention relates to compounds of formula (I) 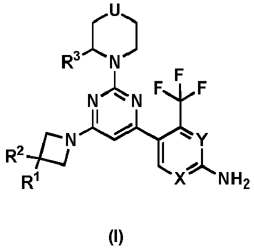 wherein
X and Y are, independently of each other CH or N;
U is O, CHOH, CHCH₂OH, C(CH₃)OH, CO, CHNH₂;
R¹ and R² are, independently of each other H, F or CH₃;
R³ is H or CH₃;
and prodrugs, metabolites, tautomers, solvates and pharmaceutically acceptable salts thereof.

Another aspect of the invention provides a pharmaceutical composition comprising a compound of formula (I) as defined hereinbefore, and a pharmaceutically acceptable carrier. The pharmaceutical composition may further comprise one or more additional therapeutic agents selected from chemotherapeutic agents, anti-proliferative agents, antiinflammatory agents, immunomodulatory agents, neurotropic factors, agents for treating blood disorders, agents for treating diabetes, and agents for treating immunodeficiency disorders.

Another aspect of the invention provides methods of inhibiting microtubule activity, comprising contacting microtubules with an effective inhibitory amount of a compound of formula (I) as defined hereinbefore.

Another aspect of the invention provides methods of preventing or treating a disease or disorder by administering to a mammal in need of such treatment an effective amount of a compound of formula (I) as defined hereinbefore. Examples of such diseases, conditions and disorders include, but are not limited to, hyperproliferative disorders (e.g., cancer, including melanoma and other cancers of the skin), neurodegeneration, cardiac hypertrophy, pain, migraine, neuro-traumatic diseases, stroke, diabetes, hepatomegaly, cardiovascular disease, Alzheimer's disease, cystic fibrosis, autoimmune diseases, atherosclerosis, restenosis, psoriasis, allergic disorders, inflammation, neurological disorders, hormone-related diseases, conditions associated with organ transplantation, immunodeficiency disorders, destructive bone disorders, hyperproliferative disorders, infectious diseases, conditions associated with cell death, thrombin-induced platelet aggregation, chronic myelogenous leukaemia (CML), liver disease, pathologic immune conditions involving T cell activation, gout, autoimmune disease, arthritis, graft rejection, inflammatory bowel disease, or proliferation induced after a medical procedure.

Another aspect of the invention provides methods of preventing or treating a hyperproliferative disorder, comprising administering to a mammal in need of such treatment an effective amount of a compound of formula (I) as defined hereinbefore, alone or in combination with one or more additional compounds having anti-hyperproliferative properties. An additional aspect of the invention is the use of a compound of this invention in the preparation of a medicament for the treatment or prevention of hyperproliferative disorders in a mammal.

Another aspect of the invention includes kits comprising a compound of formula (I) as defined hereinbefore, a container, and optionally a package insert or label indicating a treatment.

Another aspect of the invention includes methods of preparing, methods of separating, and methods of purifying compounds of formula (I), as defined hereinbefore including intermediates, prodrugs and salts of said compounds.

Another aspect of the invention includes novel intermediates useful for preparing compounds of formula (I) as defined hereinbefore.

### Detailed Description of the Invention

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments.

### Definitions

The terms "treat" and "treatment" refer to both therapeutic treatment and prophylactic or preventative measures, wherein the object is to prevent or slow down (lessen) an undesired pathological change or disorder, such as the development or spread of cancer. For purpose of this invention, beneficial or desired clinical results include, but are not limited to, alleviation of symptoms, diminishment of extent of disease, stabilized (*i.e.,* not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrase "therapeutically effective amount" means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of cancer, the therapeutically effective amount of the drug may reduce the number of cancer cells; reduce the tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can be measured, for example, by assessing the time to disease progression (TIP) and/or determining the response rate (RR).

The terms "cancer" and "cancerous" refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukaemia or lymphoid malignancies. More particular examples of such cancers include squamous cell cancer (e.g., epithelial squamous cell cancer), lung cancer including small-cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, bile duct cancer, mantle cell lymphoma, CNS lymphoma, chronic lymphocytic leukemia, non-Hodgkin's lymphoma, as well as head and neck cancer.

A "chemotherapeutic agent" is a chemical compound useful in the treatment of cancer. Examples of known chemotherapeutic agents include trastuzumab, pertuzumab, erlotinib (TARCEVA®, Genentech/Roche/OSI Pharm.), bortezomib (VELCADE®, Millennium Pharm.), fulvestrant (FASLODEX®, AstraZeneca), sunitib (SUTENT®, Pfizer/Sugen), letrozole (FEMARA®, Novartis), imatinib mesylate (GLEEVEC®, Novartis), finasunate (VATALANIB®, Novartis), oxaliplatin (ELOXATIN®, Sanofi), 5-FU (5-fluorouracil), leucovorin, rapamycin (Sirolimus, RAPAMUNE®, Wyeth), lapatinib (TYKERB®, GSK572016, Glaxo Smith Kline), lonafarnib (SCH 66336), sorafenib (NEXAVAR, Bayer Labs), and gefitinib (IRESSA®, AstraZeneca), AG1478, alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and melamines including altretamine, triethylenemelamine, triethylenephosphoramide, triethylenethiophosphoramide and trimethylomelamine; acetogenins; a camptothecin (including the synthetic analog topotecan); bryostatin; callystatin; CC-1065 (including its adozelesin, carzelesin and bizelesin synthetic analogs); cryptophycins; dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g*., calicheamicin, especially calicheamicin gammal 1 and calicheamicin omegal 1; dynemicin, including dynemicin A; biphosphonates, such as clodronate; an esperamicin; as well as neocarzinostatin chromophore and related chromoprotein enediyne antibiotic chromophores, aclacinomysins, actinomycin, authramycin, azaserine, bleomycins, cactinomycin, carabicin, carminomycin, carzinophillin, chromomycinis, dactinomycin, daunorubicin, detorubicin, 6-diazol-5-oxo-L-norleucine, ADRIAMYCIN® (doxorubicin), morpholino-doxorubicin, cyanomorpholino-doxorubicin, 2-pyrrolino-doxorubicin and deoxydoxorubicin, epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex; razoxane; rhizoxin; sizofiran; spirogermanium; tenuazonic acid; triaziquone; trichothecenes; urethane; indesine; dacarbazine; mannomustine; mitobronitol; mitolactol; pipobroman; gacytosine; arabinoside; taxoids, e.g., TAXOL® (paclitaxel; Bristol-Myers Squibb), ABRAXANE™ (Cremophor-free), albumin-engineered nanoparticle formulations of paclitaxel, and TAXOTERE® (docetaxel, doxetaxel; Sanofi-Aventis); chlorambucil; GEMZAR® (gemcitabine); 6-thioguanine; mercaptopurine; methotrexate; platinum analogs such as cisplatin and carboplatin; vinblastine; etoposide; ifosfamide; mitoxantrone; vincristine; NAVELBINE® (vinorelbine); novantrone; teniposide; edatrexate; daunomycin; aminopterin; capecitabine (XELODA®); ibandronate; CP-11; topoisomerase inhibitor RFS 2000; difluoromethylornithine (DMFO); retinoids such as retinoic acid; and pharmaceutically acceptable salts; acids and derivatives of any of the above.

Also included in the definition of "chemotherapeutic agent" are: (i) anti-hormonal agents that act to regulate or inhibit hormone action on tumors such as anti-estrogens and selective receptor modulators (SERMs), including, for example, tamoxifen (including NOLVADEX®; tamoxifen citrate), raloxifene, droloxifene, and FARESTON® (toremifine citrate); (ii) aromatase inhibitors that inhibit the enzyme aromatase, which regulates estrogen production in the adrenal glands, such as, for example, 4(5)-imidazoles, MEGASE® (megestrol acetate); AROMASIN® (exemestane; Pfizer), formestanie, fadrazole, RIVISOR® (vorozole), FEMARA® (letrozole; Novartis), and ARIMIDEX® (anastrozole; AstraZeneca); (iii) anti-androgens such as flutamide, nilutamide; (iv) protein kinase inhibitors; (v) lipid kinase inhibitors; (vi) antisense oligonucleotides, particularly those which inhibit expression of genes in signaling pathways implicated in aberrant cell proliferation, such as, for example, PKC-alpha, Raf I and H-Ras; (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN® rll-2; a topoisomerase 1 inhibitor such as LURTOTECANE®; ABARELIX® rmRH; (ix) anti-angiogenic agents such as bevacizumab (AVASTIN®, Genentech/Roche); and (x) pharmaceutically acceptable salts, acids and derivatives of any of the above.

The term "prodrug" as used in this application refers to a precursor or derivative form of a compound of the invention that may have improved properties such as better solubility, reduced cytotoxicity or increased bioavailibility compared to the parent compound or drug and is capable of being activated or converted into the more active parent form. The prodrugs of this invention include, but are not limited to, derivatives of the amino group connected to the pyridine or pyrimidine in which one or two hydrogens are replaced by a suitable substituent, or derivatitves of the hydroxyl- or aminopiperidine (where U is CHOH, CHCH₂OH, C(CH₃)OH or CHNH₂) Examples of such prodrugs are compounds acylated by an amino acid selected from the 20 most often occurring natural L-alpha-amino acids, acylated by a dipeptide such as L-Ala-L-Ala, by carbonic acid, sulfuric acid or phosphoric acid, as well as pharmaceutically acceptable salts thereof.

The phrase "pharmaceutically acceptable salt" as used herein, refers to pharmaceutically acceptable organic or inorganic salts of a compound of the invention, in particular acid addition salts. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate (mesylate), ethanesulfonate, benzenesulfonate, *p*-toluenesulfonate, and pamoate salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as *p*-toluenesulfonic acid or ethanesulfonic acid, or the like.

The phrase "pharmaceutically acceptable" indicates that the substance or composition must be compatible chemically and/or toxicologically, with the other ingredients comprising a formulation, and/or the mammal being treated therewith.

The term "protecting group" refers to a substituent that is commonly employed to block or protect a particular functionality during the reaction of other functional groups on the compound. For example, an "amino-protecting group" is a substituent attached to an amino group that blocks or protects the amino functionality in the compound. Suitable amino-protecting groups include acetyl, trifluoroacetyl, *tert*-butoxycarbonyl (BOC), benzyloxycarbonyl and 9-fluorenylmethylenoxycarbonyl (Fmoc). For a general description of protecting groups and their use, see T. W. Greene, Protective Groups in Organic Synthesis, John Wiley & Sons, New York, 1991.

The terms "compound of this invention" and "compounds of the present invention" and "compounds of formula (I)" include stereoisomers, geometric isomers, tautomers, solvates, pharmaceutically acceptable salts, and solvates of the salts thereof.

The term "mammal" includes, but is not limited to, humans, mice, rats, guinea pigs, monkeys, dogs, cats, horses, cows, pigs, and sheep.

The present invention provides 4-(azetidin-1-yl)pyrimidyl compounds with anti-mitotic and anti-proliferative activity of formula (I), and pharmaceutical formulations thereof, which are potentially useful in the treatment of hyperproliferative diseases in cancer and inflammation. More specifically, the present invention provides compounds of formula (I): 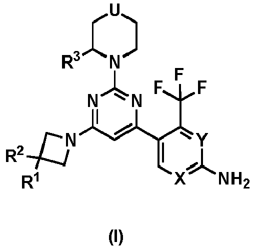 wherein
X and Y are, independently of each other CH or N;
U is O, CHOH, CHCH₂OH, C(CH₃)OH, CO, CHNH₂;
R¹ and R² are, independently of each other H, F or CH₃;
R³ is H or CH₃;
and prodrugs, metabolites, tautomers, solvates and pharmaceutically acceptable salts thereof.

Preferred are compounds wherein X is N, Y is CH and U is O.

Likewise preferred are compounds wherein X is N, Y is N and U is O.

Likewise preferred are compounds wherein X is N, Y is CH and U is CHOH.

Likewise preferred are compounds wherein X is N, Y is CH and U is CO.

Likewise preferred are compounds wherein R¹ and R² are F.

Likewise preferred are compounds wherein R¹ is H and R² is F.

Likewise preferred are compounds wherein R³ is H.

Likewise preferred are compounds wherein R³ is CH₃.

Most preferred are the following compounds shown by formula:

(The names of the corresponding structures were produced using ChemBioDraw Ultra, version 13.0.1 as well as lower and upper software versions thereof, CambridgeSoft Corp., Cambridge MA).

### Compound 1:

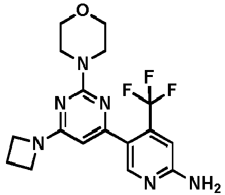

### 5-(6-(azetidin-1-yl)-2-morpholinopyrimidin-4-yl)-4-(trifluoromethyl)pyridin-2-amine

### Compound 2:

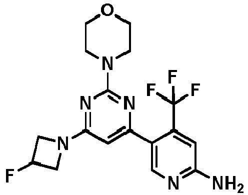

### 5-(6-(3-fluoroazetidin-1-yl)-2-morpholinopyrimidin-4-yl)-4-(trifluoromethyl)pyridin-2-amine

### Compound 3:

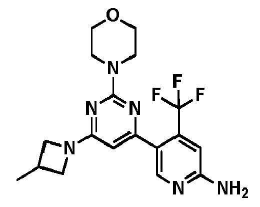

### 5-(6-(3-methylazetidin-1-yl)-2-morpholinopyrimidin-4-yl)-4-(trifluoromethyl)pyridin-2-amine

### Compound 4:

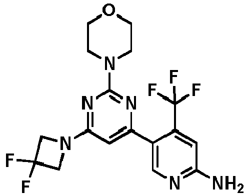

### 5-(6-(3,3-difluoroazetidin-1-yl)-2-morpholinopyrimidin-4-yl)-4-(trifluoromethyl)pyridin-2-amine

### Compound 5:

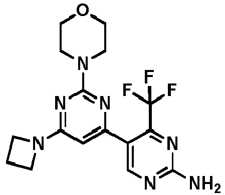

### 6-(azetidin-1-yl)-2-morpholino-4'-(trifluoromethyl)-[4,5'-bipyrimidin]-2'-amine

### Compound 6:

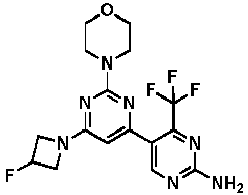

### 6-(3-fluoroazetidin-1-yl)-2-morpholino-4'-(trifluoromethyl)-[4,5'-bipyrimidin]-2'-amine

### Compound 7:

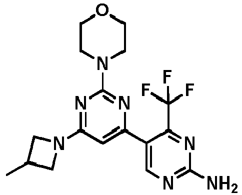

### 6-(3-methylazetidin-1-yl)-2-morpholino-4'-(trifluoromethyl)-[4,5'-bipyrimidin]-2'-amine

### Compound 8:

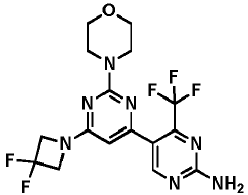

### 6-(3,3-difluoroazetidin-1-yl)-2-morpholino-4'-(trifluoromethyl)-[4,5'-bipyrimidin]-2'-amine

### Compound 9:

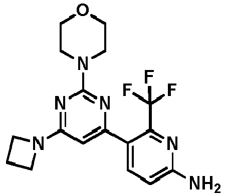

### 5-(6-(azetidin-1-yl)-2-morpholinopyrimidin-4-yl)-6-(trifluoromethyl)pyridin-2-amine

### Compound 10:

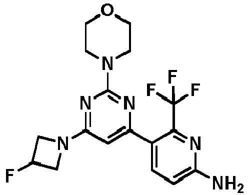

### 5-(6-(3-fluoroazetidin-1-yl)-2-morpholinopyrimidin-4-yl)-6-(trifluoromethyl)pyridin-2-amine

### Compound 11:

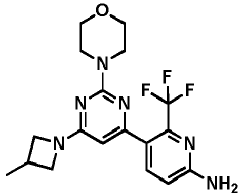

### 5-(6-(3-methylazetidin-1-yl)-2-morpholinopyrimidin-4-yl)-6-(trifluoromethyl)pyridin-2-amine

### Compound 12:

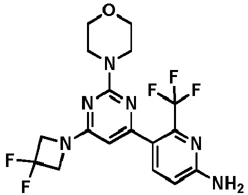

### 5-(6-(3,3-difluoroazetidin-1-yl)-2-morpholinopyrimidin-4-yl)-6-(trifluoromethyl)pyridin-2-amine

### Compound 13:

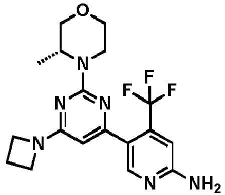

### (R)-5-(6-(azetidin-1-yl)-2-(3-methylmorpholino)pyrimidin-4-yl)-4-(trifluoromethyl)pyridin-2-amine

### Compound 14:

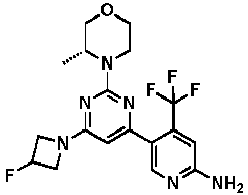

### (R)-5-(6-(3-fluoroazetidin-1-yl)-2-(3-methylmorpholino)pyrimidin-4-yl)-4-(trifluoromethyl)pyridin-2-amine

### Compound 15:

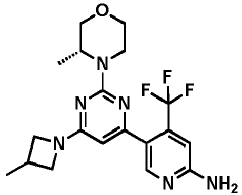

### (R)-5-(6-(3-methylazetidin-1-yl)-2-(3-methylmorpholino)pyrimidin-4-yl)-4-(trifluoromethyl)pyridin-2-amine

### Compound 16:

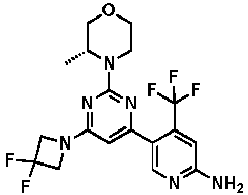

### (R)-5-(6-(3,3-difluoroazetidin-1-yl)-2-(3-methylmorpholino)pyrimidin-4-yl)-4-(trifluoromethyl)pyridin-2-amine

### Compound 17:

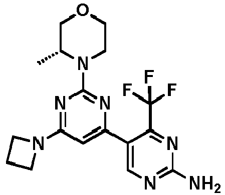

### (R)-6-(azetidin-1-yl)-2-(3-methylmorpholino)-4'-(trifluoromethyl)-[4,5'-bipyrimidin]-2'-amine

### Compound 18:

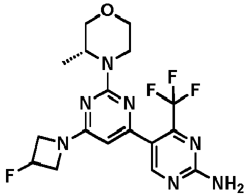

### (R)-6-(3-fluoroazetidin-1-yl)-2-(3-methylmorpholino)-4'-(trifluoromethyl)-[4,5'-bipyrimidin]-2'-amine

### Compound 19:

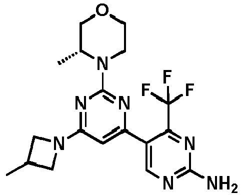

### (R)-6-(3-methylazetidin-1-yl)-2-(3-methylmorpholino)-4'-(trifluoromethyl)-[4,5'-bipyrimidin]-2'-amine

### Compound 20:

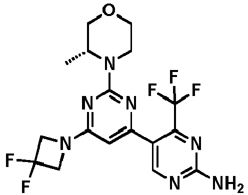

### (R)-6-(3,3-difluoroazetidin-1-yl)-2-(3-methylmorpholino)-4'-(trifluoromethyl)-[4,5'-bipyrimidin]-2'-amine

### Compound 21:

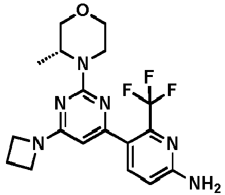

### (R)-5-(6-(azetidin-1-yl)-2-(3-methylmorpholino)pyrimidin-4-yl)-6-(trifluoromethyl)pyridin-2-amine

### Compound 22:

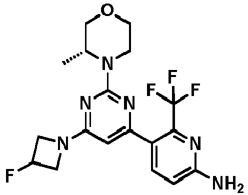

### (R)-5-(6-(3-fluoroazetidin-1-yl)-2-(3-methylmorpholino)pyrimidin-4-yl)-6-(trifluoromethyl)pyridin-2-amine

### Compound 23:

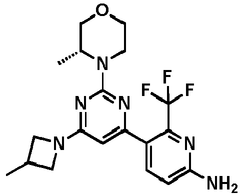

### (R)-5-(6-(3-methylazetidin-1-yl)-2-(3-methylmorpholino)pyrimidin-4-yl)-6-(trifluoromethyl)pyridin-2-amine

### Compound 24:

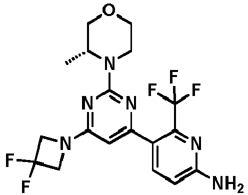

### (R)-5-(6-(3,3-difluoroazetidin-1-yl)-2-(3-methylmorpholino)pyrimidin-4-yl)-6-(trifluoromethyl)pyridin-2-amine

### Compound 25:

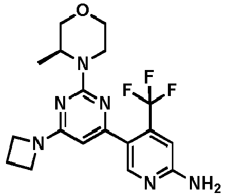

### (S)-5-(6-(azetidin-1-yl)-2-(3-methylmorpholino)pyrimidin-4-yl)-4-(trifluoromethyl)pyridin-2-amine

### Compound 26:

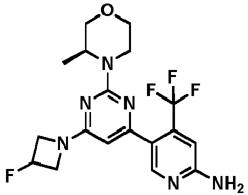

### (S)-5-(6-(3-fluoroazetidin-1-yl)-2-(3-methylmorpholino)pyrimidin-4-yl)-4-(trifluoromethyl)pyridin-2-amine

### Compound 27:

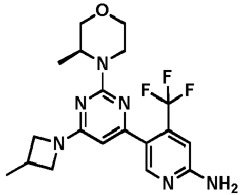

### (S)-5-(6-(3-methylazetidin-1-yl)-2-(3-methylmorpholino)pyrimidin-4-yl)-4-(trifluoromethyl)pyridin-2-amine

### Compound 28:

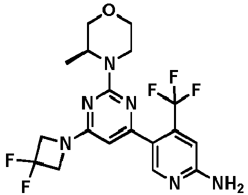

### (S)-5-(6-(3,3-difluoroazetidin-1-yl)-2-(3-methylmorpholino)pyrimidin-4-yl)-4-(trifluoromethyl)pyridin-2-amine

### Compound 29:

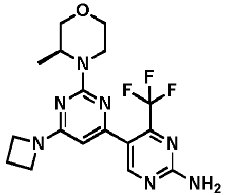

### (S)-6-(azetidin-1-yl)-2-(3-methylmorpholino)-4'-(trifluoromethyl)-[4,5'-bipyrimidin]-2'-amine

### Compound 30:

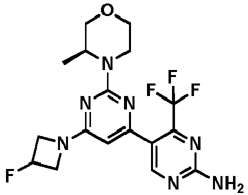

### (S)-6-(3-fluoroazetidin-1-yl)-2-(3-methylmorpholino)-4'-(trifluoromethyl)-[4,5'-bipyrimidin]-2'-amine

### Compound 31:

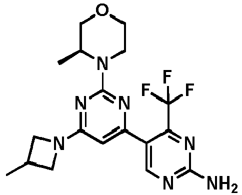

### (S)-6-(3-methylazetidin-1-yl)-2-(3-methylmorpholino)-4'-(trifluoromethyl)-[4,5'-bipyrimidin]-2'-amine

### Compound 32:

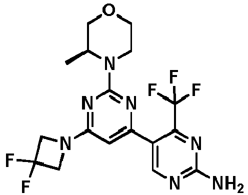

### (S)-6-(3,3-difluoroazetidin-1-yl)-2-(3-methylmorpholino)-4'-(trifluoromethyl)-[4,5'-bipyrimidin]-2'-amine

### Compound 33:

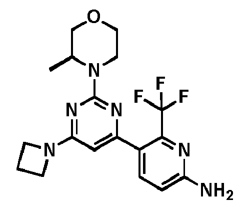

### (S)-5-(6-(azetidin-1-yl)-2-(3-methylmorpholino)pyrimidin-4-yl)-6-(trifluoromethyl)pyridin-2-amine

### Compound 34:

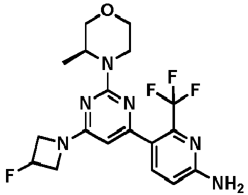

### (S)-5-(6-(3-fluoroazetidin-1-yl)-2-(3-methylmorpholino)pyrimidin-4-yl)-6-(trifluoromethyl)pyridin-2-amine

### Compound 35:

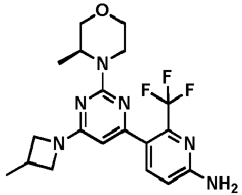

### (S)-5-(6-(3-methylazetidin-1-yl)-2-(3-methylmorpholino)pyrimidin-4-yl)-6-(trifluoromethyl)pyridin-2-amine

### Compound 36:

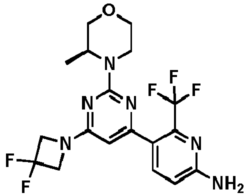

### (S)-5-(6-(3,3-difluoroazetidin-1-yl)-2-(3-methylmorpholino)pyrimidin-4-yl)-6-(trifluoromethyl)pyridin-2-amine

### Compound 37:

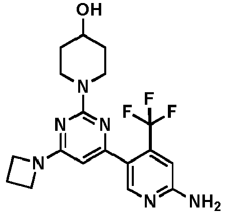

### 1-(4-(6-amino-4-(trifluoromethyl)pyridin-3-yl)-6-(azetidin-1-yl)pyrimidin-2-yl)piperidin-4-ol

### Compound 38:

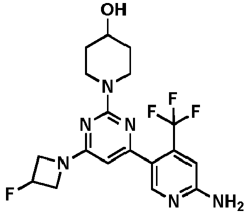

### 1-(4-(6-amino-4-(trifluoromethyl)pyridin-3-yl)-6-(3-fluoroazetidin-1-yl)pyrimidin-2-yl)piperidin-4-ol

### Compound 39:

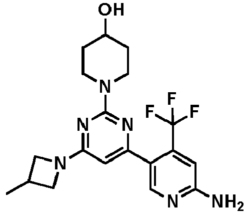

### 1-(4-(6-amino-4-(trifluoromethyl)pyridin-3-yl)-6-(3-methylazetidin-1-yl)pyrimidin-2-yl)piperidin-4-ol

### Compound 40:

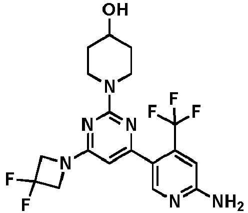

### 1-(4-(6-amino-4-(trifluoromethyl)pyridin-3-yl)-6-(3,3-difluoroazetidin-1-yl)pyrimidin-2-yl)piperidin-4-ol

### Compound 41:

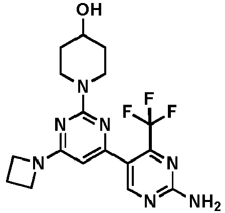

### 1-(2'-amino-6-(azetidin-1-yl)-4'-(trifluoromethyl)-[4,5'-bipyrimidin]-2-yl)piperidin-4-ol

### Compound 42:

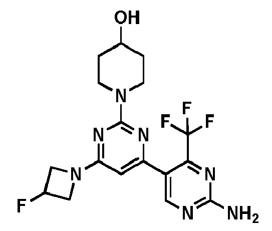

### 1-(2'-amino-6-(3-fluoroazetidin-1-yl)-4'-(trifluoromethyl)-[4,5'-bipyrimidin]-2-yl)piperidin-4-ol

### Compound 43:

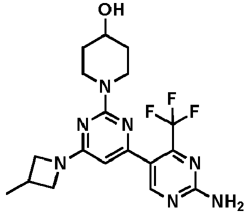

### 1-(2'-amino-6-(3-methylazetidin-1-yl)-4'-(trifluoromethyl)-[4,5'-bipyrimidin]-2-yl)piperidin-4-ol

### Compound 44:

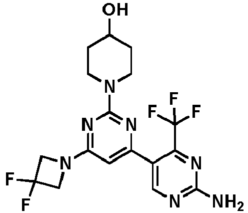

### 1-(2'-amino-6-(3,3-difluoroazetidin-1-yl)-4'-(trifluoromethyl)-[4,5'-bipyrimidin]-2-yl)piperidin-4-ol

### Compound 45:

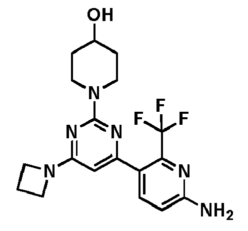

### 1-(4-(6-amino-2-(trifluoromethyl)pyridin-3-yl)-6-(azetidin-1-yl)pyrimidin-2-yl)piperidin-4-ol

### Compound 46:

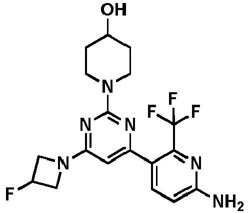

### 1-(4-(6-amino-2-(trifluoromethyl)pyridin-3-yl)-6-(3-fluoroazetidin-1-yl)pyrimidin-2-yl)piperidin-4-ol

### Compound 47:

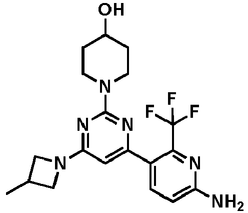

### 1-(4-(6-amino-2-(trifluoromethyl)pyridin-3-yl)-6-(3-methylazetidin-1-yl)pyrimidin-2-yl)piperidin-4-ol

### Compound 48:

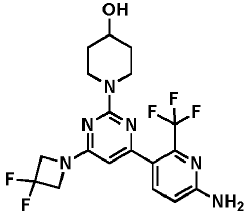

### 1-(4-(6-amino-2-(trifluoromethyl)pyridin-3-yl)-6-(3,3-difluoroazetidin-1-yl)pyrimidin-2-yl)piperidin-4-ol

### Compound 49:

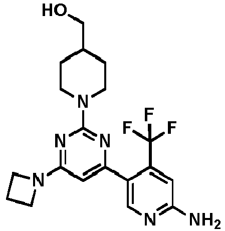

### (1-(4-(6-amino-4-(trifluoromethyl)pyridin-3-yl)-6-(azetidin-1-yl)pyrimidin-2-yl)piperidin-4-yl)methanol

### Compound 50:

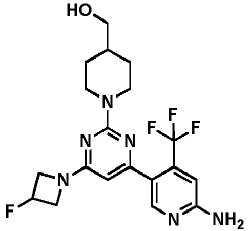

### (1-(4-(6-amino-4-(trifluoromethyl)pyridin-3-yl)-6-(3-fluoroazetidin-1-yl)pyrimidin-2-yl)piperidin-4-yl)methanol

### Compound 51:

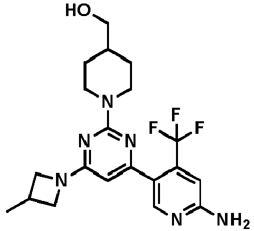

### (1-(4-(6-amino-4-(trifluoromethyl)pyridin-3-yl)-6-(3-methylazetidin-1-yl)pyrimidin-2-yl)piperidin-4-yl)methanol

### Compound 52:

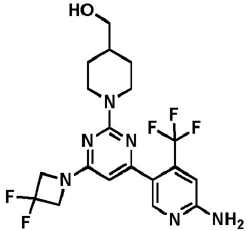

### (1-(4-(6-amino-4-(trifluoromethyl)pyridin-3-yl)-6-(3,3-difluoroazetidin-1-yl)pyrimidin-2-yl)piperidin-4-yl)methanol

### Compound 53:

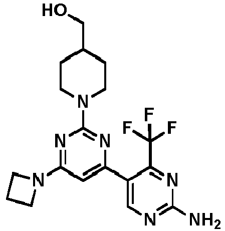

### (1-(2'-amino-6-(azetidin-1-yl)-4'-(trifluoromethyl)-[4,5'-bipyrimidin]-2-yl)piperidin-4-yl)methanol

### Compound 54:

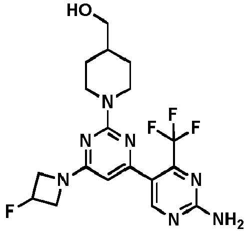

### (1-(2'-amino-6-(3-fluoroazetidin-1-yl)-4'-(trifluoromethyl)-[4,5'-bipyrimidin]-2-yl)piperidin-4-yl)methanol

### Compound 55:

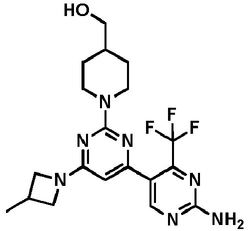

### (1-(2'-amino-6-(3-methylazetidin-1-yl)-4'-(trifluoromethyl)-[4,5'-bipyrimidin]-2-yl)piperidin-4-yl)methanol

### Compound 56:

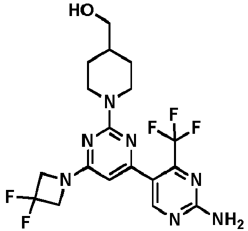

### (1-(2'-amino-6-(3,3-difluoroazetidin-1-yl)-4'-(trifluoromethyl)-[4,5'-bipyrimidin]-2-yl)piperidin-4-yl)methanol

### Compound 57:

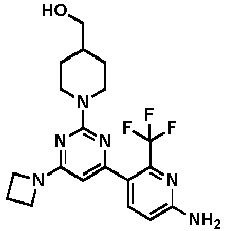

### (1-(4-(6-amino-2-(trifluoromethyl)pyridin-3-yl)-6-(azetidin-1-yl)pyrimidin-2-yl)piperidin-4-yl)methanol

### Compound 58:

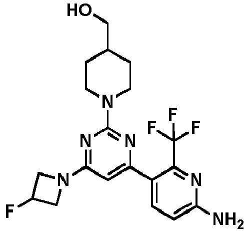

### (1-(4-(6-amino-2-(trifluoromethyl)pyridin-3-yl)-6-(3-fluoroazetidin-1-yl)pyrimidin-2-yl)piperidin-4-yl)methanol

### Compound 59:

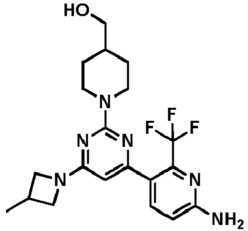

### (1-(4-(6-amino-2-(trifluoromethyl)pyridin-3-yl)-6-(3-methylazetidin-1-yl)pyrimidin-2-yl)piperidin-4-yl)methanol

### Compound 60:

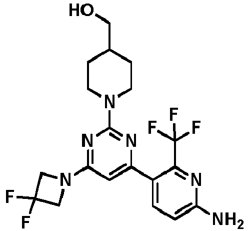

### (1-(4-(6-amino-2-(trifluoromethyl)pyridin-3-yl)-6-(3,3-difluoroazetidin-1-yl)pyrimidin-2-yl)piperidin-4-yl)methanol

### Compound 61:

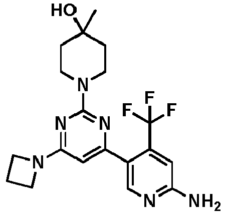

### 1-(4-(6-amino-4-(trifluoromethyl)pyridin-3-yl)-6-(azetidin-1-yl)pyrimidin-2-yl)-4-methylpiperidin-4-ol

### Compound 62:

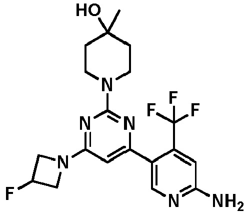

### 1-(4-(6-amino-4-(trifluoromethyl)pyridin-3-yl)-6-(3-fluoroazetidin-1-yl)pyrimidin-2-yl)-4-methylpiperidin-4-ol

### Compound 63:

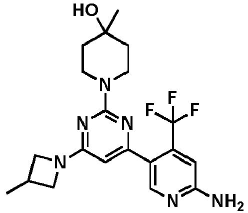

### 1-(4-(6-amino-4-(trifluoromethyl)pyridin-3-yl)-6-(3-methylazetidin-1-yl)pyrimidin-2-yl)-4-methylpiperidin-4-ol

### Compound 64:

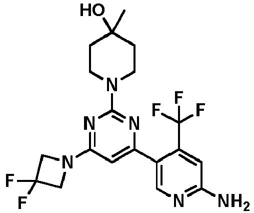

### 1-(4-(6-amino-4-(trifluoromethyl)pyridin-3-yl)-6-(3,3-difluoroazetidin-1-yl)pyrimidin-2-yl)-4-methylpiperidin-4-ol

### Compound 65:

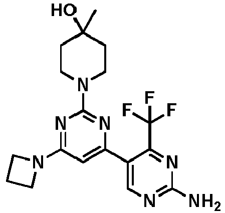

### 1-(2'-amino-6-(azetidin-1-yl)-4'-(trifluoromethyl)-[4,5'-bipyrimidin]-2-yl)-4-methylpiperidin-4-ol

### Compound 66:

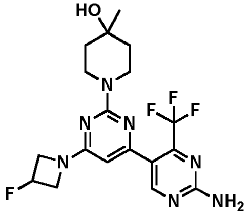

### 1-(2'-amino-6-(3-fluoroazetidin-1-yl)-4'-(trifluoromethyl)-[4,5'-bipyrimidin]-2-yl)-4-methylpiperidin-4-ol

### Compound 67:

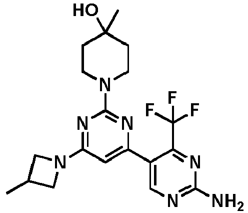

### 1-(2'-amino-6-(3-methylazetidin-1-yl)-4'-(trifluoromethyl)-[4,5'-bipyrimidin]-2-yl)-4-methylpiperidin-4-ol

### Compound 68:

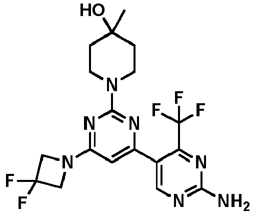

### 1-(2'-amino-6-(3,3-difluoroazetidin-1-yl)-4'-(trifluoromethyl)-[4,5'-bipyrimidin]-2-yl)-4-methylpiperidin-4-ol

### Compound 69:

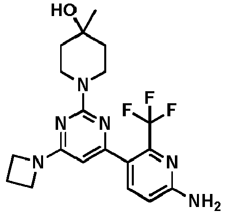

### 1-(4-(6-amino-2-(trifluoromethyl)pyridin-3-yl)-6-(azetidin-1-yl)pyrimidin-2-yl)-4-methylpiperidin-4-ol

### Compound 70:

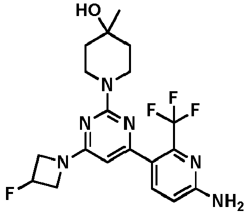

### 1-(4-(6-amino-2-(trifluoromethyl)pyridin-3-yl)-6-(3-fluoroazetidin-1-yl)pyrimidin-2-yl)-4-methylpiperidin-4-ol

### Compound 71:

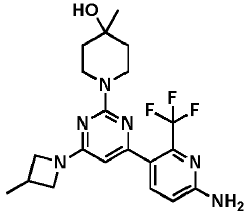

### 1-(4-(6-amino-2-(trifluoromethyl)pyridin-3-yl)-6-(3-methylazetidin-1-yl)pyrimidin-2-yl)-4-methylpiperidin-4-ol

### Compound 72:

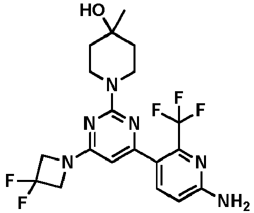

### 1-(4-(6-amino-2-(trifluoromethyl)pyridin-3-yl)-6-(3,3-difluoroazetidin-1-yl)pyrimidin-2-yl)-4-methylpiperidin-4-ol

### Compound 73:

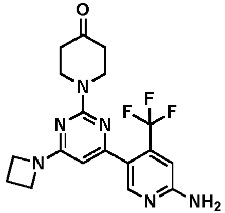

### 1-(4-(6-amino-4-(trifluoromethyl)pyridin-3-yl)-6-(azetidin-1-yl)pyrimidin-2-yl)piperidin-4-one

### Compound 74:

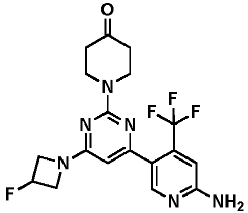

### 1-(4-(6-amino-4-(trifluoromethyl)pyridin-3-yl)-6-(3-fluoroazetidin-1-yl)pyrimidin-2-yl)piperidin-4-one

### Compound 75:

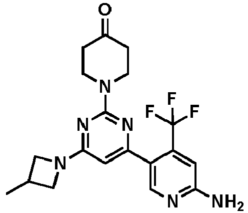

### 1-(4-(6-amino-4-(trifluoromethyl)pyridin-3-yl)-6-(3-methylazetidin-1-yl)pyrimidin-2-yl)piperidin-4-one

### Compound 76:

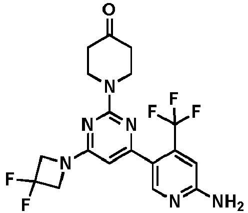

### 1-(4-(6-amino-4-(trifluoromethyl)pyridin-3-yl)-6-(3,3-difluoroazetidin-1-yl)pyrimidin-2-yl)piperidin-4-one

### Compound 77:

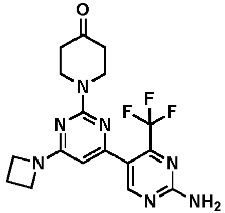

### 1-(2'-amino-6-(azetidin-1-yl)-4'-(trifluoromethyl)-[4,5'-bipyrimidin]-2-yl)piperidin-4-one

### Compound 78:

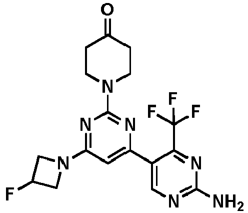

### 1-(2'-amino-6-(3-fluoroazetidin-1-yl)-4'-(trifluoromethyl)-[4,5'-bipyrimidin]-2-yl)piperidin-4-one

### Compound 79:

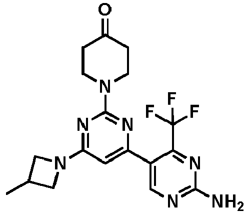

### 1-(2'-amino-6-(3-methylazetidin-1-yl)-4'-(trifluoromethyl)-[4,5'-bipyrimidin]-2-yl)piperidin-4-one

### Compound 80:

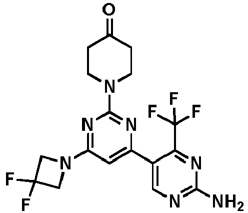

### 1-(2'-amino-6-(3,3-difluoroazetidin-1-yl)-4'-(trifluoromethyl)-[4,5'-bipyrimidin]-2-yl)piperidin-4-one

### Compound 81:

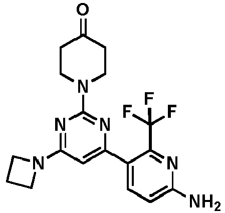

### 1-(4-(6-amino-2-(trifluoromethyl)pyridin-3-yl)-6-(azetidin-1-yl)pyrimidin-2-yl)piperidin-4-one

### Compound 82:

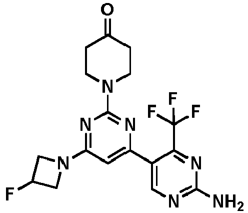

### 1-(2'-amino-6-(3-fluoroazetidin-1-yl)-4'-(trifluoromethyl)-[4,5'-bipyrimidin]-2-yl)piperidin-4-one

### Compound 83:

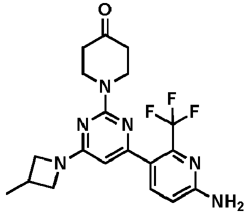

### 1-(4-(6-amino-2-(trifluoromethyl)pyridin-3-yl)-6-(3-methylazetidin-1-yl)pyrimidin-2-yl)piperidin-4-one

### Compound 84:

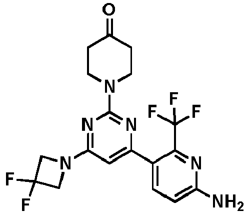

### 1-(4-(6-amino-2-(trifluoromethyl)pyridin-3-yl)-6-(3,3-difluoroazetidin-1-yl)pyrimidin-2-yl)piperidin-4-one

### Compound 85:

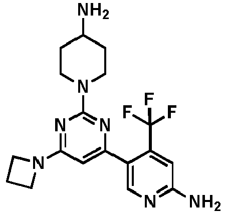

### 5-(2-(4-aminopiperidin-1-yl)-6-(azetidin-1-yl)pyrimidin-4-yl)-4-(trifluoromethyl)pyridin-2-amine

### Compound 86:

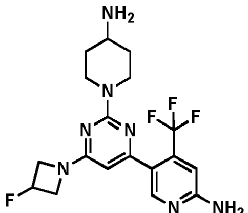

### 5-(2-(4-aminopiperidin-1-yl)-6-(3-fluoroazetidin-1-yl)pyrimidin-4-yl)-4-(trifluoromethyl)pyridin-2-amine

### Compound 87:

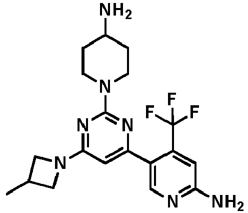

### 5-(2-(4-aminopiperidin-1-yl)-6-(3-methylazetidin-1-yl)pyrimidin-4-yl)-4-(trifluoromethyl)pyridin-2-amine

### Compound 88:

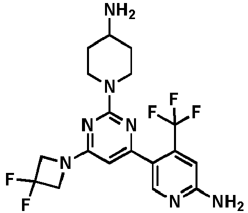

### 5-(2-(4-aminopiperidin-1-yl)-6-(3,3-difluoroazetidin-1-yl)pyrimidin-4-yl)-4-(trifluoromethyl)pyridin-2-amine

### Compound 89:

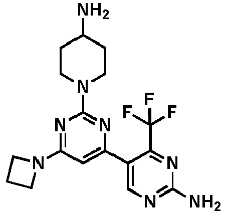

### 2-(4-aminopiperidin-1-yl)-6-(azetidin-1-yl)-4'-(trifluoromethyl)-[4,5'-bipyrimidin]-2'-amine

### Compound 90:

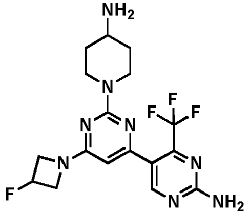

### 2-(4-aminopiperidin-1-yl)-6-(3-fluoroazetidin-1-yl)-4'-(trifluoromethyl)-[4,5'-bipyrimidin]-2'-amine

### Compound 91:

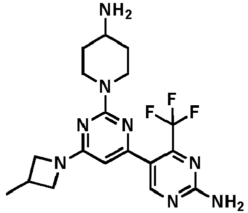

### 2-(4-aminopiperidin-1-yl)-6-(3-methylazetidin-1-yl)-4'-(trifluoromethyl)-[4,5'-bipyrimidin]-2'-amine

### Compound 92:

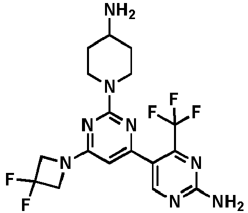

### 2-(4-aminopiperidin-1-yl)-6-(3,3-difluoroazetidin-1-yl)-4'-(trifluoromethyl)-[4,5'-bipyrimidin]-2'-amine

### Compound 93:

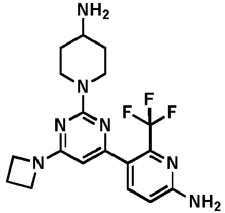

### 5-(2-(4-aminopiperidin-1-yl)-6-(azetidin-1-yl)pyrimidin-4-yl)-6-(trifluoromethyl)pyridin-2-amine

### Compound 94:

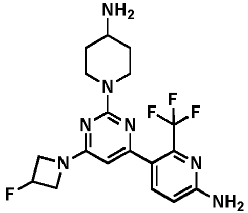

### 5-(2-(4-aminopiperidin-1-yl)-6-(3-fluoroazetidin-1-yl)pyrimidin-4-yl)-6-(trifluoromethyl)pyridin-2-amine

### Compound 95:

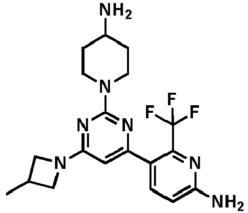

### 5-(2-(4-aminopiperidin-1-yl)-6-(3-methylazetidin-1-yl)pyrimidin-4-yl)-6-(trifluoromethyl)pyridin-2-amine

### Compound 96:

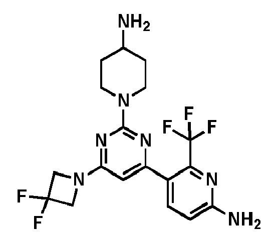

### 5-(2-(4-aminopiperidin-1-yl)-6-(3,3-difluoroazetidin-1-yl)pyrimidin-4-yl)-6-(trifluoromethyl)pyridin-2-amine

### Preparation of compounds of the invention

The compounds of the invention may be synthesized by synthetic routes that include processes analogous to those well known in the chemical arts, particularly in light of the description contained herein. The starting materials are generally available from commercial sources or are readily prepared using methods well known to those skilled in the art.

In preparing compounds of the invention, protection of remote functionality (e.g., primary or secondary amine) of intermediates may be necessary. The need for such protection will vary depending on the nature of the remote functionality and the conditions of the preparation methods. Suitable amino-protecting groups include *tert*-butyloxycarbonyl (BOC), *tert*-butyldimethylsilyl (TMS) or *N,N*-dimethylformamidinyl. The need for such protection is readily determined by one skilled in the art. For a general description of protecting groups and their use, see T. W. Greene, Protective Groups in Organic Synthesis, John Wiley & Sons, New York, 1991.

### Methods of separation

In the methods of preparing the compounds of this invention, it may be advantageous to separate reaction products from one another and/or from starting materials. The desired products of each step or series of steps are separated and/or purified to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; high, medium and low pressure liquid chromatography methods and apparatus; small scale analytical; and preparative thin or thick layer chromatography, as well as techniques of small scale thin layer and flash chromatography.

Selection of appropriate methods of separation depends on the nature of the materials involved, for example, presence or absence of polar functional groups in chromatography, stability of materials in acidic and basic media in multiphase extraction, and the like. One skilled in the art will apply techniques most likely to achieve the desired separation.

### Examples

The Examples are intended to illustrate the present invention without restricting it.

The chemical reactions described in the Examples may be readily adapted to prepare a number of other microtubule inhibitors of the invention, and alternative methods for preparing the compounds of this invention are deemed to be within the scope of this invention. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds of the invention.

As a rule, ¹H NMR and mass spectra have been obtained for the compounds prepared. In the Examples described below, unless otherwise indicated, all temperatures are set forth in degrees Celsius (°C). Reagents were purchased from commercial suppliers such as Sigma Aldrich, Fluorochem, Acros, Lancaster, TCI or Maybridge, and were used without further purification unless otherwise indicated. The reactions set forth below were done generally under a positive pressure of nitrogen or with a drying tube (unless otherwise stated) in anhydrous solvents, and the reaction flasks were typically fitted with rubber septa for the introduction of substrates and reagents *via* syringe. Glassware was oven dried. Column chromatography was performed using Merck silica gel. ¹H NMR spectra were recorded on a Bruker instrument operating at 400 MHz. ¹H NMR spectra were obtained for solutions in various deuterated solvents such as CDCl₃ and (CD₃)₂SO. The chemical shift *δ* values were reported in ppm and corrected to the signal of the deuterated solvents (7.26 ppm for CDCl₃ and 2.50 ppm for (CD₃)₂SO) or TMS (0.00 ppm). ¹⁹F NMR spectra were calibrated relative to CFCl₃ (δ=0 ppm) as external standard. ¹⁹F NMR spectra were recorded ¹H-decoupled. When peak multiplicities are reported, the following abbreviations are used: s (singlet), d (doublet), t (triplet), m (multiplet), quint. (quintet), br (broadened). Coupling constants, when given, are reported in Hertz (Hz). MALDI-ToF Mass spectra (MS) have been obtained on a Voyager-DeTM Pro measured in *m*/*z.*

The following abbreviations are used hereinafter: BSA (bovine serum albumine), DMSO (dimethyl sulfoxide), HCl (hydrochloric acid), M (molar), MALDI (Matrix-assisted Laser Desorption/lonization), MS (mass spectrometry), PBS (phosphate buffered saline), TLC (thin layer chromatography).

### Preparation of Intermediate Compounds:

The following methods were used to prepare the intermediates compounds used to produce compounds of formula (I).

### Method 1: 4-(4-chloro-6-(3,3-difluoroazetidin-1-yl)pyrimidin-2-yl)morpholine (I1)

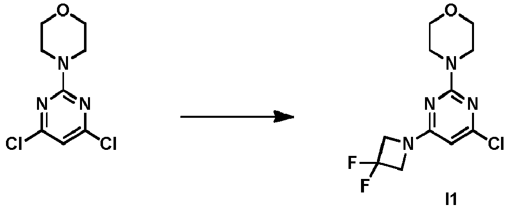

4-(4,6-Dichloropyrimidin-2-yl)morpholine (1.59 g, 6.80 mmol, 1.0 eq.) and 3,3-difluoroazetidine hydrochloride (968 mg, 7.45 mmol, 1.1 eq.) are mixed in dioxane (41 mL). *N,N*-Diisopropylethylamine (3.54 mL, 20.4 mmol, 3.0 eq.) is added and the resulting mixture is heated at reflux for 16 hours (the conversion is monitored by TLC analysis). The mixture is reduced to dryness under reduced pressure and the residue is purified by column chromatography on silica gel (cyclohexane / ethyl acetate 1:0 → 7:3). The desired intermediate **I1** is obtained as a colorless solid (93% yield). ¹H NMR (400 MHz, CDCl₃): δ5.66 (s, 1 H), 4.34 (t, ³*J*_{H,F} = 12 Hz, 4 H), 3.78-3.69 (m, 8 H); ¹⁹F{¹H} NMR (376 MHz, CDCl₃): δ - 99.9 (s, 2 F); MS (MALDI): *m*/*z* = 291.2 ([M+H]⁺).

### Method 1 is also used for the preparation of the following intermediate compounds I2, I3 and I4.

| | Reagent | Structure | NMR | MS |
|---|---|---|---|---|
| **I2** | 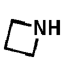 | 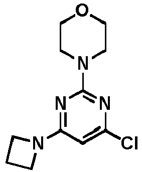 | ¹H NMR (400 MHz, CDCl₃): *δ* 5.54 (s, 1 H), 4.02 (t, ³*J*_{H,H} = 7.5 Hz, 4 H), 3.76-3.68 (m, 8 H), 2.38 (quint., ³*J*_{H,H} = 7.5 Hz, 2 H). | MS (MALDI): *m*/*z* = 255.2 ([M+H]⁺). |
| **I3** | 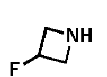 | 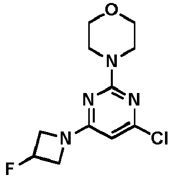 | ¹H NMR (400 MHz, CDCl₃): δ5.60 (s, 1 H), 5.51-5.03 (m, 1 H), 4.35-4.23 (m, 4 H), 3.77-3.69 (m, 8 H); ¹⁹F{¹H} NMR (376 MHz, CDCl₃): δ - 180.2 (s, 1 F). | |
| **I4** | 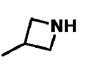 | 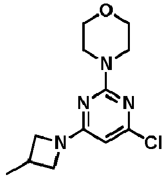 | ¹H NMR (400 MHz, CDCl₃): δ 5.54 (s, 1 H), 4.12 (t, ³*J*_{H,H} = 8.3 Hz, 2 H), 3.76-3.68 (m, 8 H), 3.60-3.53 (m, 2 H), 2.88-2.75 (m, 1 H), 1.29 (d, ³*J*_{H,H} = 6.9 Hz, 3 H). | |

### Method 2: 2,4-dichloro-6-(3,3-difluoroazetidin-1-yl)pyrimidine (I5)

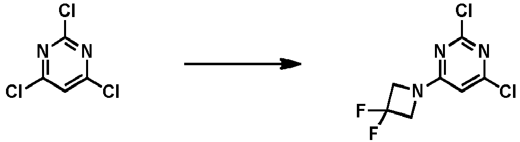

Under nitrogen atmosphere, 2,4,6-trichloropyrimidine (1.00 g, 5.46 mmol, 1.0 eq.) and 3,3-difluoroazetidine hydrochloride (708 mg, 5.46 mmol, 1.0 eq.) is dissolved in dichloromethane (5.0 mL). The mixture is cooled to 0°C and triethylamine (1.66mL, 12.0 mmol, 2.2 eq.) is added dropwise over a period of 1 hour. The reaction mixture is then stirred for 2 hours, where it is allowed to warm to room temperature. The resulting suspension is diluted with dichloromethane (100 mL) and is stirred vigorously for further 24 hours at room temperature. Deionized H₂O is added, the layers are separated and the aqueous layer is extracted with dichloromethane (3 x). The combined organic layer is washed with deionized H₂O (1 x), brine (1 x), dried over anhydrous sodium sulfate, filtered and the solvent is evaporated under reduced pressure. Purification by column chromatography on silica gel (cyclohexane / ethyl acetate 1:0→3:1) gives the desired intermediate **I5** as a colorless solid (75% yield). ¹H NMR (400 MHz, CDCl₃): δ6.22 (s, 1 H), 4.48 (t, ³*J*_{H,F} = 12 Hz, 4 H); ¹⁹F{¹H} NMR (376 MHz, CDCl₃): δ - 99.8 (s, 2 F).

### Method 3: (R)-4-(4-chloro-6-(3,3-difluoroazetidin-1-yl)pyrimidin-2-yl)-3-methylmorpholine (I6)

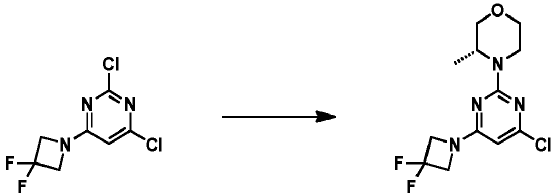

2,4-Dichloro-6-(3,3-difluoroazetidin-1-yl)pyrimidine (I**5,** 150 mg, 625 µmol, 1.0 eq.), (R)-3-methylmorpholine (63.0 mg, 623 µmol, 1.0 eq.) and *N,N*-diisopropylethylamine (0.400 mL, 2.29 mmol, 3.7 eq.) are mixed in dioxane (4 mL). The resulting mixture is heated at 100 °C overnight. The conversion is monitor by TLC analysis. The reaction mixture is then cooled to room temperature and the solvent is evaporated under reduced pressure. The resulting residue is purified by column chromatography on silica gel (cyclohexane / ethyl acetate 1:0 → 9:1). The desired intermediate **I6** is obtained as a colorless solid (48% yield). ¹H NMR (400 MHz, CDCl₃): δ5.65 (s, 1 H), 4.70-4.60 (m, 1 H), 4.88-4.24 (m, 5 H), 3.93 (dd, *²J_{H,H} =* 11 Hz, ³*J*_{H,H} *=* 3.7 Hz, 1 H), 3.73 (d, *²J_{H,H} =* 11 Hz, 1 H), 3.65 (dd, *²J_{H,H} =* 11 Hz, ³*J*_{H,H} *=* 3.2 Hz, 1 H), 3.53-3.45 (m, 1 H), 3.27-3.17 (m, 1 H), 1.28 (d, *³J_{H,H}* = 6.9 Hz, 3 H); ¹⁹F{¹H} NMR (376 MHz, CDCl₃): δ - 99.9 (s, 2 F).

### Method 3 is also used for the preparation of the following intermediate compounds I7 and I8.

| | Reagent | Structure | NMR | MS |
|---|---|---|---|---|
| **I7** | 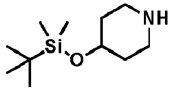 | 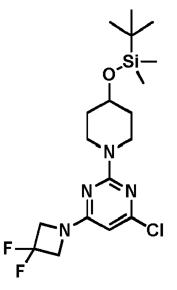 | ¹H NMR (400 MHz, CDCl₃): δ 5.46 (s, 1 H), 4.19 (t, *³J_{H,F}* = 12Hz, 4H), 3.95-3.77 (m, 3 H), 3.48-3.42 (m, 2 H), 1.64-1.57 (m, 2 H), 1.43-1.35 (m, 2 H), 0.76 (s, 9 H), - 0.07 (s, 6 H); ¹⁹F{¹_{H}} NMR (376 MHz, CDCl₃): δ - 99.9 (s, 2 F). | MS (MALDI): *m*/*z =* 419.4 ([M+H]⁺). |
| **I8** | 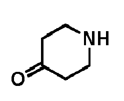 | 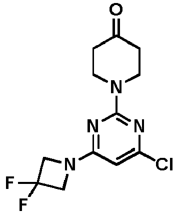 | ¹H NMR (400 MHz, CDCl₃): δ 5.71 (s, 1 H), 4.37 (t, *³J_{H,F}* = 12 Hz, 4 H), 4.07 (t, *³J_{H,H} =* 8.1 Hz, 4 H), 2.48 (t, *³J_{H,H} =* 8.1Hz, 4 H); ¹⁹F{¹H} NMR (376 MHz, CDCl₃): δ - 99.8 (s, 2 F). | MS (MALDI): *m*/*z =* 303.2 ([M+H]⁺). |

### Method 4: N'-(5-bromo-4-(trifluoromethyl)pyridin-2-yl)-N,N-dimethylformimidamide (I9)

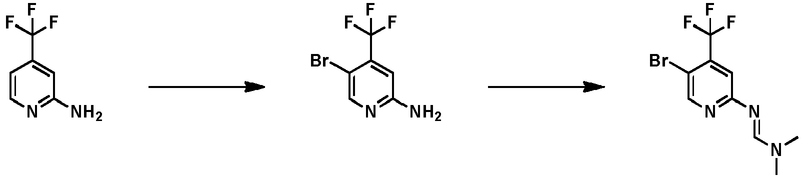

*N*-Bromosuccinimide (29.2 g, 164 mmol, 1.1 eq.) was slowly added to a solution of 2-amino-6-(trifluoromethyl)pyridine (25.0 g, 156 mmol, 1.0 eq.) in tetrahydrofuran (250 mL) at 0 °C. The resulting mixture was allowed to stir at room temperature for 15 hours. Then, *N,N*-dimethylformamide dimethyl acetal (25.1 mL, 188 mmol, 1.2 eq.) was added and the mixture was heated at 70 °C for 6 hours. The solvent was oratory evaporated under reduced pressure and the residue was recrystallized from hexane to yield the desired product **I9** as an off-white solid (65% yield). ¹H NMR (400 MHz, CDCl₃): *δ* 8.46 (s, 1 H), 8.38 (s, 1 H), 7.18 (s, 1 H), 3.11 (s, 3 H), 3.08 (s, 3 H); ¹⁹F{¹H} NMR (376 MHz, CDCl₃): *δ* - 65.0 (s, 3 F).

### Method 5: N,N-dimethyl-N'-(5-(4,4,5,5-tetramethyl-1,3,2-dioxaborolan-2-yl)-4-(trifluoro methyl)pyridin-2-yl)formimidamide (I10)

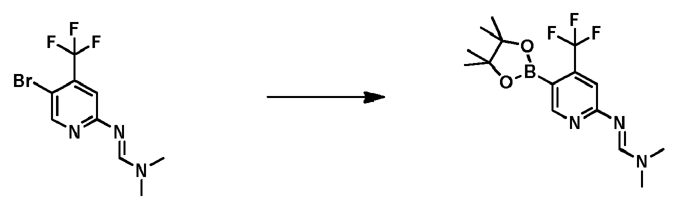

To a solution of *N*'-(5-Bromo-4-(trifluoromethyl)pyridin-2-yl)-*N,N*-dimethylformimidamide **(I9,** 20.0 g, 67.6 mmol, 1.0 eq.) in tetrahydrofuran (140 mL) at 0 °C, was slowly added a solution of isopropylmagnesium chloride in tetrahydrofuran (2 M, 38.9 mL, 77.8 mmol, 1.2 eq.). The resulting yellow suspension was stirred at room temperature for 1 hour. Then, 2-isopropoxy-4,4,5,5-tetramethyl-1,3,2-dioxaborolane (19.1 mL, 94.6 mmol, 1.4 eq.) was added and the reaction mixture was placed in a preheated oil bath at 65 °C. After 3 hours, the reaction mixture was cooled down to room temperature and poured onto an aqueous saturated NH₄Cl-solution. The resulting layers were separated and the organic layer was evaporated under reduced pressure. The residue was then dissolved in hexane and washed with an aqueous saturated NaHCO₃-solution (2 x). The organic layer was dried over anhydrous Na₂SO₄, filtered and the solvent was evaporated under reduced pressure. The residue was recrystallized from hexane to yield the desired compound **I10** as a yellowish solid (40% yield). ¹H NMR (400 MHz, CDCl₃): *δ* 8.58 (s, 1 H), 8.51 (s, 1 H), 7.14 (s, 1 H), 3.09 (s, 3 H), 3.08 (s, 3 H), 1.32 (s, 12 H); ¹⁹F{¹H} NMR (376 MHz, CDCl₃): δ - 62.7 (s, 3 F).

### Preparation of Compounds of the Invention

### General procedure 1:

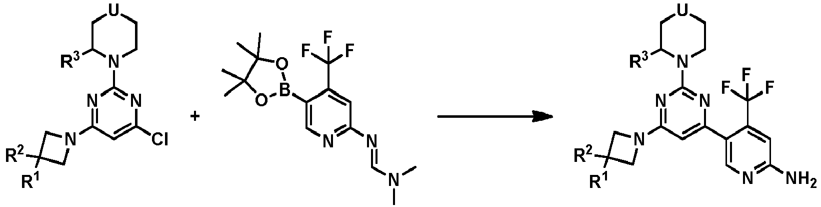

Substituted monochloro-pyrimidine (1.0 eq.), compound **I10** (1.1 eq.), potassium phosphate tribasic (2.0 eq.) and chloro(2-dicyclohexylphosphino-2',4',6'-triisopropyl-1,1'-biphenyl) [2-(2'-amino-1,1'-biphenyl)]-palladium(II) (Sigma-Aldrich, product number 741825, 0.05 eq.) are charged in a flask. Under N₂-atmosphere, dioxane (30 volumes) and deionized water (1.5 volume) are added and the resulting mixture is then directly placed into an oil bath pre-heated at 95 °C. The reaction mixture is stirred at this temperature for 2 hours.
A 5 M aqueous HCl-solution (20 eq.) is added. The resulting mixture is heated to 60 °C overnight. The mixture is then allowed to cool down to room temperature, the pH is adjusted to 8-9 by addition of a 2 M aqueous solution of sodium hydroxide and the mixture is extracted with ethyl acetate (3 x 20 volumes). The combined organic layers are dried over anhydrous sodium sulfate, filtered and the solvent is evaporated under reduced pressure. Purification by column chromatography on silica gel affords the desired product.

### General procedure 2:

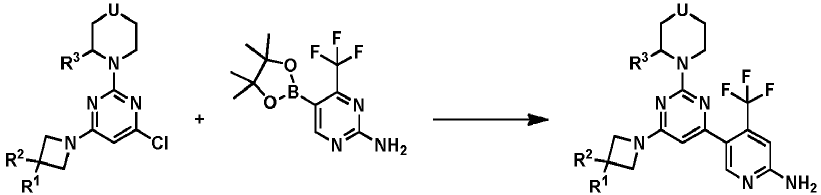

Substituted monochloro-pyrimidine (1.0 eq.), 5-(4,4,5,5-tetramethyl-1,3,2-dioxaborolan-2-yl)-4-(trifluoromethyl)pyrimidin-2-amine (1.1 eq.), potassium phosphate tribasic (2.0 eq.) and chloro(2-dicyclohexylphosphino-2',4',6'-triisopropyl-1,1'-biphenyl) [2-(2'-amino-1,1'-biphenyl)]-palladium(II) (Sigma-Aldrich, product number 741825, 0.05 eq.) are charged in a flask. Under N₂-atmosphere, dioxane (30 volumes) and deionized water (1.5 volume) are added and the resulting mixture is then directly placed into an oil bath pre-heated at 95 °C. The reaction mixture is stirred at this temperature for 2-6 hours. After this time, 2 M aqueous solution of sodium hydroxide is added and the mixture is then extracted with ethyl acetate (3 x 20 volumes). The combined organic layers are dried over anhydrous sodium sulfate, filtered and the solvent is evaporated under reduced pressure. Purification by column chromatography on silica gel affords the desired product.

### Example 1:

### 5-(6-(azetidin-1-yl)-2-morpholinopyrimidin-4-yl)-4-(trifluoromethyl)pyridin-2-amine (1)

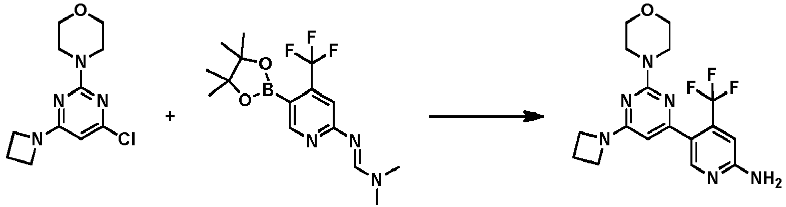

According to general procedure 1, compound 1 is obtained from starting materials **I2** and **I10** in 81% yield as a colorless solid. ¹H NMR (400 MHz, CDCl₃): *δ* 8.25 (s, 1 H), 6.73 (s, 1 H), 5.66 (s, 1 H), 5.08 (br s, 2 H), 4.06 (t, ³*J*_{H,H} = 7.4 Hz, 4 H), 3.80-3.70 (m, 8 H), 2.38 (quint., ³*J*_{H,H} *=* 7.4 Hz, 2 H); ¹⁹F{¹H} NMR (376 MHz, CDCl₃): *δ* - 59.7 (s, 3 F); MS (MALDI): *m*/*z* = 381.5 ([M+H]⁺).

### Example 2:

### 5-(6-(3-fluoroazetidin-1-yl)-2-morpholinopyrimidin-4-yl)-4-(trifluoromethyl)pyridin-2-amine (2)

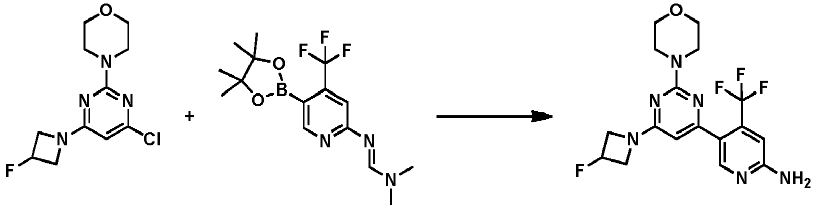

According to general procedure 1, compound **2** is obtained from starting materials **I3** and **I10** in 52% yield as a colorless solid. ¹H NMR (400 MHz, CDCl₃): *δ* 8.26 (s, 1 H), 6.78 (s, 1 H), 5.71 (s, 1 H), 5.53-5.33 (m, 1 H), 4.77 (br s, 2 H), 4.39-4.27 (m, 2 H), 4.22-4.09 (m, 2 H), 3.82-3.69 (m, 8 H); ¹⁹F{¹H} NMR (376 MHz, CDCl₃): *δ* - 59.7 (s, 3 F), - 180.2 (s, 1 F); MS (MALDI): *m*/*z* = 399.2 ([M+H]⁺).

### Example 3:

### 5-(6-(3-methylazetidin-1-yl)-2-morpholinopyrimidin-4-yl)-4-(trifluoromethyl)pyridin-2-amine (3)

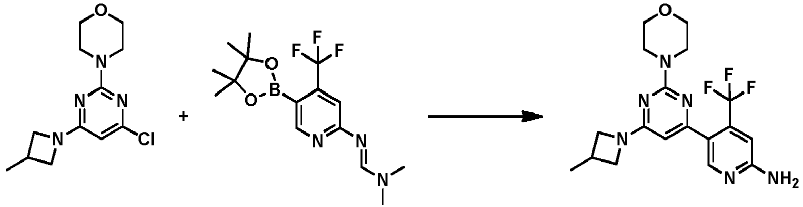

According to general procedure 1, compound **3** is obtained from starting materials **I4** and **I10** in 67% yield as a colorless solid. ¹H NMR (400 MHz, CDCl₃): *δ* 8.27 (s, 1 H), 6.77 (s, 1 H), 5.65 (s, 1 H), 4.73 (br s, 2 H), 4.15 (t, ³*J*_{H,H} *=* 8.2 Hz, 2 H), 3.80-3.70 (m, 8 H), 3.63-3.57 (m, 2 H), 2.89-2.77 (m, 1 H), 1.30 (d, *³J_{H,H} =* 6.9 Hz, 3 H); ¹⁹F{¹H} NMR (376 MHz, CDCl₃): *δ* - 59.8 (s, 3 F); MS (MALDI): *mlz* = 395.2 ([M+H]⁺).

### Example 4:

### 5-(6-(3,3-difluoroazetidin-1-yl)-2-morpholinopyrimidin-4-yl)-4-(trifluoromethyl)pyridin-2-amine (4)

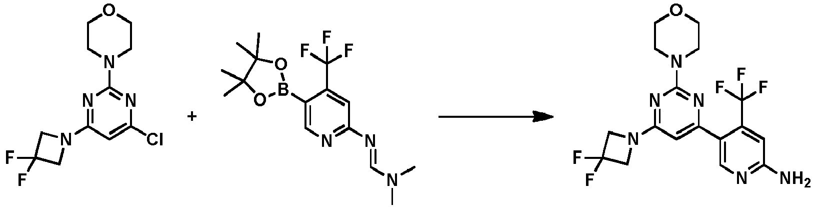

According to general procedure 1, compound **4** is obtained from starting materials **I1** and **I10** in 84% yield as a colorless solid. ¹H NMR (400 MHz, CDCl₃): *δ* 8.18 (s, 1 H), 6.82 (s, 1 H), 6.78 (s, 2 H), 6.01 (s, 1 H), 4.45 (t, *³J_{H,F} =* 12 Hz, 4 H), 3.69-3.58 (m, 8 H); ¹⁹F{¹H} NMR (376 MHz, CDCl₃): *δ* - 58.7 (s, 3 F), - 98.9 (s, 2 F); MS (MALDI): *m*/*z =* 417.0 ([M+H]⁺).

### Example 5:

### 6-(3,3-difluoroazetidin-1-yl)-2-morpholino-4'-(trifluoromethyl)-[4,5'-bipyrimidin]-2'-amine (8)

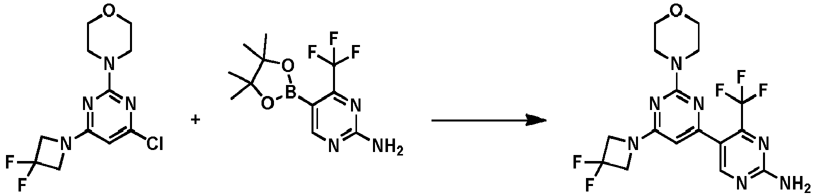

According to general procedure 2, compound **8** is obtained from starting materials **I1** and 5-(4,4,5,5-tetramethyl-1,3,2-dioxaborolan-2-yl)-4-(trifluoromethyl)pyrimidin-2-amine in 88% yield as a colorless solid. ¹H NMR (400 MHz, CDCl₃): *δ* 8.57 (s, 1 H), 5.75 (s, 1 H), 5.37 (br s, 2 H), 4.38 (t, *³J_{H,H} =* 12 Hz, 4 H), 3.82-3.72 (m, 8 H); ¹⁹F{¹H} NMR (376 MHz, CDCl₃): *δ* - 64.5 (s, 3 F), - 99.8 (s, 2 F); MS (MALDI): *m*/*z* = 418.2 ([M+H]⁺).

### Example 6:

### (R)-5-(6-(3,3-difluoroazetidin-1-yl)-2-(3-methylmorpholino)pyrimidin-4-yl)-4-(trifluoromethyl)pyridin-2-amine (16)

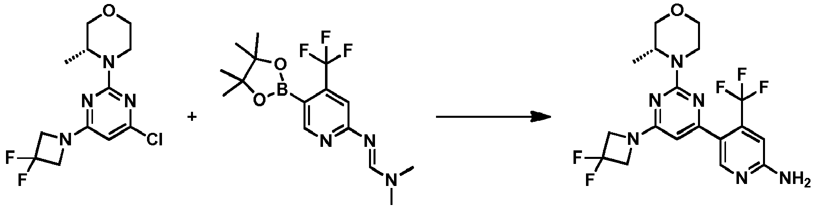

According to general procedure 1, compound **16** is obtained from starting materials **I6** and **I10** in 70% yield as a colorless solid. ¹H NMR (400 MHz, CDCl₃): *δ* 8.26 (s, 1 H), 6.79 (s, 1 H), 5.75 (s, 1 H), 4.82-4.69 (m, 3 H), 4.43-4.30 (m, 5 H), 3.97-3.90 (m, 1 H), 3.76-3.64 (m, 2 H), 3.57-3.48 (m, 1 H), 3.28-3.17 (m, 1 H), 1.27 (d, ³*J*_{H,H} *=* 6.8 Hz, 3 H); ¹⁹F{¹H} NMR (376 MHz, CDCl₃): *δ* - 59.8 (s, 3 F), - 99.8 (s, 2 F); MS (MALDI): *m*/*z =* 431.3 ([M+H]⁺).

### Example 7:

### 1-(4-(6-amino-4-(trifluoromethyl)pyridin-3-yl)-6-(3,3-difluoroazetidin-1-yl)pyrimidin-2-yl)piperidin-4-ol (40)

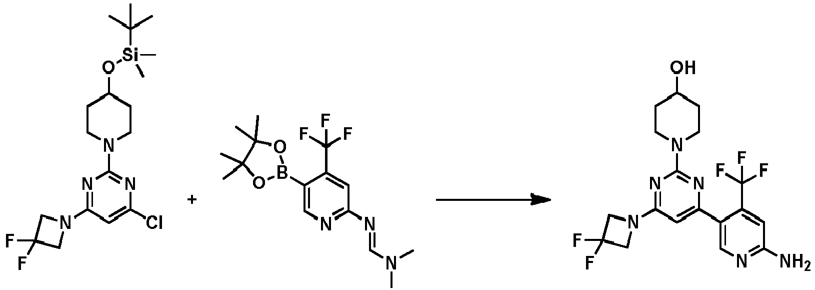

According to general procedure 1, compound **40** is obtained from starting materials **I7** and **I10** in 71% yield as a colorless solid. ¹H NMR (400 MHz, (CD₃)₂SO): *δ* 8.17 (s, 1 H), 6.82 (s, 1 H), 6.76 (br s, 2 H), 5.93 (s, 1 H), 4.68 (d, ³*J*_{H,H} *=* 4.3 Hz, 1 H), 4.43 (t, *³J_{H,F} =* 12 Hz, 4 H), 4.32-4.24 (m, 2 H), 3.73-3.64 (m, 1 H), 3.19-3.10 (m, 2 H), 1.78-1.68 (m, 2 H), 1.33-1.21 (m, 2 H); ¹⁹F{¹H} NMR (376 MHz, (CD₃)₂SO): *δ* - 58.7 (s, 3 F), - 98.9 (s, 2 F); MS (MALDI): *m*/*z* = 431.2 ([M+H]⁺).

### Example 8:

### 1-(4-(6-amino-4-(trifluoromethyl)pyridin-3-yl)-6-(3,3-difluoroazetidin-1-yl)pyrimidin-2-yl)piperidin-4-one (76)

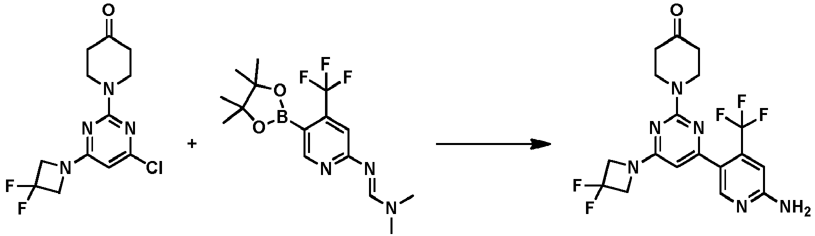

According to general procedure 1, compound 76 is obtained from starting materials **I8** and **I10** in 31% yield as a colorless solid. ¹H NMR (400 MHz, (CD₃)₂SO): *δ* 8.21 (s, 1 H), 6.83 (s, 1 H), 6.79 (br s, 2 H), 6.05 (s, 1 H), 4.47 (t, *³J_{H,F} =* 12 Hz, 4 H), 4.01 (t, *³J_{H,H} =* 6.1 Hz, 4 H), 2.35 (t, ³*J*_{H,H} *=* 6.0 Hz, 4 H); ¹⁹F{¹H} NMR (376 MHz, (CD₃)₂SO): *δ* - 58.7 (s, 3 F), - 98.9 (s, 2 F); MS (MALDI): *mlz* = 429.3 ([M+H]⁺).

### Determination of microtubule binding in melanoma cells

Microtubule binding of compounds of formula (I) was determined in A2058 melanoma cells measuring mitotic arrest in high-throughput, high-content microscopic approaches. Microtubule networks or their depolymerization were visualized in parallel. Cellular tests are beneficial to address the biological question of tubulin binding, but also indirectly measure the uptake of a compound by cells. A hallmark of microtubule modifying agents (stabilizing or de-stabilizing) is a mitotic arrest cell cycle arrest of dividing cells, which was measured using the following protocol:
*Cell preparation:* 5000 A2058 cells were seeded in 100 µl complete DMEM into 96-well plates (Cell Carrier, Perkin Elmer) the day before inhibitor treatment. Cells were exposed to inhibitors (ranging from 1 - 0.008 µM) for 24 hours and fixed by addition of half a volume 10% PFA in PBS (30 min., RT). Cells were permeabilized and stained with Hoechst33324 by addition of 1:10 volume of 1%BSA/1% Triton X-100, 10 µM Hoechst33324 in PBS (30 min., RT), followed by a first image acquisition. Subsequently cells were additionally stained with rabbit anti pSer¹⁰ Histone H3 (Cell Signaling Technologies) and rat anti-α-tubulin (Santa Cruz Technologies, overnight, 4 °C, shaking). Cells were washed and incubated with Alexa 488 conjugated goat anti rat IgG, Alexa 647 conjugated goat anti rabbit IgG antibodies (Life Technologies) and 1 µM Hoechst33324 in 1% BSA/0.1% Triton X-100/1x PBS (shaking; RT; dark), followed by 3 wash cycles and a 2^{nd} round of imaging.
*Image acquisition:* Fluorescent microscopy images were acquired on an Operetta high content analysis system (Perkin Elmer). 35 fields of view/well were acquired as wide field images (20xWD objective). 1^{st} *acquisition:* Nuclear DNA content and DNA morphology was imaged using the DAPI filter set. 2^{nd} *acquisition:* Nuclear DNA, phosphorylated Histone H3 and α-tubulin images were acquired using DAPI, Alexa488 and Alexa647 filter sets.
*Image analysis:* Images were batch analysed using Columbus software (PerkinElmer). Analyses were run including single cell analysis mode. The total number of nuclei was determined in the DAPI channel. Nuclear DNA was classified into "normal" or "condensed" using a linear classifier mode. The percentage of cells with condensed DNA was calculated for each well (% condensed = (# condensed/ # total) *100).
The fraction of cells with pSer10 Histone H3 positive nuclei was determined using Hoechst33342 and Alexa647 signals. 1^{st} the nuclei were identified using DNA staining (Hoechst33342; excluding nuclei at image borders). 2^{nd} the identified nuclei were classified positive or negative for pSer10 Histone H3 by two filters using the "filter by properties" mode based on Alexa 647 intensities. Alexa 647 filter properties were *i*) mean intensities above threshold /nucleus and *ii*) a maximum intensity above threshold. Calculation: % Alexa 647^{positive} = (# Alexa 647^{positive} / # selected nuclei) *100.

The percentage of cells with condensed DNA and the percentage of pSer10 Histone H3 positive nuclei were blotted as function of inhibitor concentration (log scale) using GraphPad Prism. Half maximal effective concentrations (EC₅₀) of inhibitors were determined using GraphPad's "non-linear regression with variable slope" formula (Y=Bottom + (Top-Bottom)/(1+10^((LogEC50-X)*HillSlope))). Results are collected in Table 1.

**Table 1: Effect of selected formula (I) compounds on mitotic arrest in A2058 cells. Half-maximal effective concentrations are given as mean value of at least four experiments for the indicated cellular phenotypes.**

| | **Half-maximal effective concentrations (EC₅₀), [nM]** | |
|---|---|---|
| | **Condensed DNA** | **pSer10 Histone H3** |
| Compound **1** | 79 | 80 |
| Compound **2** | 71 | 151 |
| Compound **3** | 92 | n.d. |
| Compound **4** | 65 | 79 |
| Compound **8** | 70 | 73 |
| Compound **16** | 111 | 115 |
| Compound **40** | 128 | 135 |
| Compound **76** | 129 | 127 |

## Claims

1. A compound of formula (I), 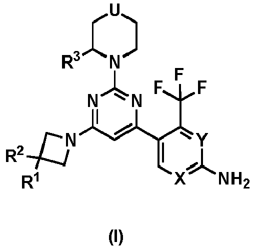 wherein
X and Y are, independently of each other CH or N;
U is O, CHOH, CHCH₂OH, C(CH₃)OH, CO, CHNH₂;
R¹ and R² are, independently of each other H, F or CH₃;
R³ is H or CH₃;
and prodrugs, metabolites, tautomers, solvates and pharmaceutically acceptable salts thereof.

2. The compound of formula (I) according to claim 1, wherein X and Y are N; and tautomers, solvates and pharmaceutically acceptable salts thereof.

3. The compound of formula (I) according to claim 1, wherein X is N and Y is CH; and tautomers, solvates and pharmaceutically acceptable salts thereof.

4. The compound of formula (I) according to any one of the claims 1 to 3, wherein R¹ is F and R² is F; and tautomers, solvates and pharmaceutically acceptable salts thereof.

5. The compound of formula (I) according to any one of the claims 1 to 3, wherein R¹ is H and R² is F; and tautomers, solvates and pharmaceutically acceptable salts thereof.

6. The compound of formula (I) according to any one of the claims 1 to 3, wherein R¹ is H and R² is H; and tautomers, solvates and pharmaceutically acceptable salts thereof.

7. The compound of formula (I) according to any one of the claims 1 to 3, wherein R¹ is H and R² is CH₃; and tautomers, solvates and pharmaceutically acceptable salts thereof.

8. The compound of formula (I) according to any one of the claims 1 to 7, wherein R³ is H; and tautomers, solvates and pharmaceutically acceptable salts thereof.

9. The compound of formula (I) according to any one of the claims 1 to 7, wherein R³ is CH₃; and tautomers, solvates and pharmaceutically acceptable salts thereof.

10. The compound of formula (I) according to any one of the claims 1 to 9, wherein U is O, CHOH or CO; and tautomers, solvates and pharmaceutically acceptable salts thereof.

11. The compound of formula (I) according to claim 3, wherein
(i) U is O, R³ is H, R¹ is H and R² is F;
(ii) U is O, R³ is H, R¹ is F and R² is F;
(iii) U is O, R³ is CH₃, R¹ is H and R² is F; or
(iv) U is O, R³ is CH₃, R¹ is F and R² is F;
and tautomers, solvates and pharmaceutically acceptable salts thereof.

12. The compound of formula (I) according to claim 1, selected from the group consisting of
- 5-(6-(azetidin-1-yl)-2-morpholinopyrimidin-4-yl)-4-(trifluoromethyl)pyridin-2-amine
- 5-(6-(3-fluoroazetidin-1-yl)-2-morpholinopyrimidin-4-yl)-4-(trifluoromethyl)pyridin-2-amine
- 5-(6-(3-methylazetidin-1-yl)-2-morpholinopyrimidin-4-yl)-4-(trifluoromethyl)pyridin-2-amine
- 5-(6-(3,3-difluoroazetidin-1-yl)-2-morpholinopyrimidin-4-yl)-4-(trifluoromethyl)pyridin-2-amine
- 6-(azetidin-1-yl)-2-morpholino-4'-(trifluoromethyl)-[4,5'-bipyrimidin]-2'-amine
- 6-(3-fluoroazetidin-1-yl)-2-morpholino-4'-(trifluoromethyl)-[4,5'-bipyrimidin]-2'-amine
- 6-(3-methylazetidin-1-yl)-2-morpholino-4'-(trifluoromethyl)-[4,5'-bipyrimidin]-2'-amine
- 6-(3,3-difluoroazetidin-1-yl)-2-morpholino-4'-(trifluoromethyl)-[4,5'-bipyrimidin]-2'-amine
- 5-(6-(azetidin-1-yl)-2-morpholinopyrimidin-4-yl)-6-(trifluoromethyl)pyridin-2-amine
- 5-(6-(3-fluoroazetidin-1-yl)-2-morpholinopyrimidin-4-yl)-6-(trifluoromethyl)pyridin-2-amine
- 5-(6-(3-methylazetidin-1-yl)-2-morpholinopyrimidin-4-yl)-6-(trifluoromethyl)pyridin-2-amine
- 5-(6-(3,3-difluoroazetidin-1-yl)-2-morpholinopyrimidin-4-yl)-6-(trifluoromethyl)pyridin-2-amine
- (*R*)-5-(6-(azetidin-1-yl)-2-(3-methylmorpholino)pyrimidin-4-yl)-4-(trifluoromethyl)pyridin-2-amine
- (*R*)-5-(6-(3-fluoroazetidin-1-yl)-2-(3-methylmorpholino)pyrimidin-4-yl)-4-(trifluoromethyl)pyridin-2-amine
- (*R*)-5-(6-(3-methylazetidin-1-yl)-2-(3-methylmorpholino)pyrimidin-4-yl)-4-(trifluoromethyl)pyridin-2-amine
- (*R*)-5-(6-(3,3-difluoroazetidin-1-yl)-2-(3-methylmorpholino)pyrimidin-4-yl)-4-(trifluoromethyl)pyridin-2-amine
- (*R*)-6-(azetidin-1-yl)-2-(3-methylmorpholino)-4'-(trifluoromethyl)-[4,5'-bipyrimidin]-2'-amine
- (*R*)-6-(3-fluoroazetidin-1-yl)-2-(3-methylmorpholino)-4'-(trifluoromethyl)-[4,5'-bipyrimidin]-2'-amine
- (*R*)-6-(3-methylazetidin-1-yl)-2-(3-methylmorpholino)-4'-(trifluoromethyl)-[4,5'-bipyrimidin]-2'-amine
- (*R*)-6-(3,3-difluoroazetidin-1-yl)-2-(3-methylmorpholino)-4'-(trifluoromethyl)-[4,5'-bipyrimidin]-2'-amine
- (*R*)-5-(6-(azetidin-1-yl)-2-(3-methylmorpholino)pyrimidin-4-yl)-6-(trifluoromethyl)pyridin-2-amine
- (*R*)-5-(6-(3-fluoroazetidin-1-yl)-2-(3-methylmorpholino)pyrimidin-4-yl)-6-(trifluoromethyl)pyridin-2-amine
- (*R*)-5-(6-(3-methylazetidin-1-yl)-2-(3-methylmorpholino)pyrimidin-4-yl)-6-(trifluoromethyl)pyridin-2-amine
- (*R*)-5-(6-(3,3-difluoroazetidin-1-yl)-2-(3-methylmorpholino)pyrimidin-4-yl)-6-(trifluoromethyl)pyridin-2-amine
- (*S*)-5-(6-(azetidin-1-yl)-2-(3-methylmorpholino)pyrimidin-4-yl)-4-(trifluoromethyl)pyridin-2-amine
- (*S*)-5-(6-(3-fluoroazetidin-1-yl)-2-(3-methylmorpholino)pyrimidin-4-yl)-4-(trifluoromethyl)pyridin-2-amine
- (*S*)-5-(6-(3-methylazetidin-1-yl)-2-(3-methylmorpholino)pyrimidin-4-yl)-4-(trifluoromethyl)pyridin-2-amine
- (*S*)-5-(6-(3,3-difluoroazetidin-1-yl)-2-(3-methylmorpholino)pyrimidin-4-yl)-4-(trifluoromethyl)pyridin-2-amine
- (*S*)-6-(azetidin-1-yl)-2-(3-methylmorpholino)-4'-(trifluoromethyl)-[4,5'-bipyrimidin]-2'-amine
- (*S*)-6-(3-fluoroazetidin-1-yl)-2-(3-methylmorpholino)-4'-(trifluoromethyl)-[4,5'-bipyrimidin]-2'-amine
- (*S*)-6-(3-methylazetidin-1-yl)-2-(3-methylmorpholino)-4'-(trifluoromethyl)-[4,5'-bipyrimidin]-2'-amine
- (*S*)-6-(3,3-difluoroazetidin-1-yl)-2-(3-methylmorpholino)-4'-(trifluoromethyl)-[4,5'-bipyrimidin]-2'-amine
- (*S*)-5-(6-(azetidin-1-yl)-2-(3-methylmorpholino)pyrimidin-4-yl)-6-(trifluoromethyl)pyridin-2-amine
- (*S*)-5-(6-(3-fluoroazetidin-1-yl)-2-(3-methylmorpholino)pyrimidin-4-yl)-6-(trifluoromethyl)pyridin-2-amine
- (*S*)-5-(6-(3-methylazetidin-1-yl)-2-(3-methylmorpholino)pyrimidin-4-yl)-6-(trifluoromethyl)pyridin-2-amine
- (S)-5-(6-(3,3-difluoroazetidin-1-yl)-2-(3-methylmorpholino)pyrimidin-4-yl)-6-(trifluoromethyl)pyridin-2-amine
- 1-(4-(6-amino-4-(trifluoromethyl)pyridin-3-yl)-6-(azetidin-1-yl)pyrimidin-2-yl)piperidin-4-ol
- 1-(4-(6-amino-4-(trifluoromethyl)pyridin-3-yl)-6-(3-fluoroazetidin-1-yl)pyrimidin-2-yl)piperidin-4-ol
- 1-(4-(6-amino-4-(trifluoromethyl)pyridin-3-yl)-6-(3-methylazetidin-1-yl)pyrimidin-2-yl)piperidin-4-ol
- 1-(4-(6-amino-4-(trifluoromethyl)pyridin-3-yl)-6-(3,3-difluoroazetidin-1-yl)pyrimidin-2-yl)piperidin-4-ol
- 1-(2'-amino-6-(azetidin-1-yl)-4'-(trifluoromethyl)-[4,5'-bipyrimidin]-2-yl)piperidin-4-ol
- 1-(2'-amino-6-(3-fluoroazetidin-1-yl)-4'-(trifluoromethyl)-[4,5'-bipyrimidin]-2-yl)piperidin-4-ol
- 1-(2'-amino-6-(3-methylazetidin-1-yl)-4'-(trifluoromethyl)-[4,5'-bipyrimidin]-2-yl)piperidin-4-ol
- 1-(2'-amino-6-(3,3-difluoroazetidin-1-yl)-4'-(trifluoromethyl)-[4,5'-bipyrimidin]-2-yl)piperidin-4-ol
- 1-(4-(6-amino-2-(trifluoromethyl)pyridin-3-yl)-6-(azetidin-1-yl)pyrimidin-2-yl)piperidin-4-ol
- 1-(4-(6-amino-2-(trifluoromethyl)pyridin-3-yl)-6-(3-fluoroazetidin-1-yl)pyrimidin-2-yl)piperidin-4-ol
- 1-(4-(6-amino-2-(trifluoromethyl)pyridin-3-yl)-6-(3-methylazetidin-1-yl)pyrimidin-2-yl)piperidin-4-ol
- 1-(4-(6-amino-2-(trifluoromethyl)pyridin-3-yl)-6-(3,3-difluoroazetidin-1-yl)pyrimidin-2-yl)piperidin-4-ol
- (1-(4-(6-amino-4-(trifluoromethyl)pyridin-3-yl)-6-(azetidin-1-yl)pyrimidin-2-yl)piperidin-4-yl)methanol
- (1-(4-(6-amino-4-(trifluoromethyl)pyridin-3-yl)-6-(3-fluoroazetidin-1-yl)pyrimidin-2-yl)piperidin-4-yl)methanol
- (1-(4-(6-amino-4-(trifluoromethyl)pyridin-3-yl)-6-(3-methylazetidin-1-yl)pyrimidin-2-yl)piperidin-4-yl)methanol
- (1-(4-(6-amino-4-(trifluoromethyl)pyridin-3-yl)-6-(3,3-difluoroazetidin-1-yl)pyrimidin-2-yl)piperidin-4-yl)methanol
- (1-(2'-amino-6-(azetidin-1-yl)-4'-(trifluoromethyl)-[4,5'-bipyrimidin]-2-yl)piperidin-4-yl)methanol
- (1-(2'-amino-6-(3-fluoroazetidin-1-yl)-4'-(trifluoromethyl)-[4,5'-bipyrimidin]-2-yl)piperidin-4-yl)methanol
- (1-(2'-amino-6-(3-methylazetidin-1-yl)-4'-(trifluoromethyl)-[4,5'-bipyrimidin]-2-yl)piperidin-4-yl)methanol
- (1-(2'-amino-6-(3,3-difluoroazetidin-1-yl)-4'-(trifluoromethyl)-[4,5'-bipyrimidin]-2-yl)piperidin-4-yl)methanol
- (1-(4-(6-amino-2-(trifluoromethyl)pyridin-3-yl)-6-(azetidin-1-yl)pyrimidin-2-yl)piperidin-4-yl)methanol
- (1-(4-(6-amino-2-(trifluoromethyl)pyridin-3-yl)-6-(3-fluoroazetidin-1-yl)pyrimidin-2-yl)piperidin-4-yl)methanol
- (1-(4-(6-amino-2-(trifluoromethyl)pyridin-3-yl)-6-(3-methylazetidin-1-yl)pyrimidin-2-yl)piperidin-4-yl)methanol
- (1-(4-(6-amino-2-(trifluoromethyl)pyridin-3-yl)-6-(3,3-difluoroazetidin-1-yl)pyrimidin-2-yl)piperidin-4-yl)methanol
- 1-(4-(6-amino-4-(trifluoromethyl)pyridin-3-yl)-6-(azetidin-1-yl)pyrimidin-2-yl)-4-methylpiperidin-4-ol
- 1-(4-(6-amino-4-(trifluoromethyl)pyridin-3-yl)-6-(3-fluoroazetidin-1-yl)pyrimidin-2-yl)-4-methylpiperidin-4-ol
- 1-(4-(6-amino-4-(trifluoromethyl)pyridin-3-yl)-6-(3-methylazetidin-1-yl)pyrimidin-2-yl)-4-methylpiperidin-4-ol
- 1-(4-(6-amino-4-(trifluoromethyl)pyridin-3-yl)-6-(3,3-difluoroazetidin-1-yl)pyrimidin-2-yl)-4-methylpiperidin-4-ol
- 1-(2'-amino-6-(azetidin-1-yl)-4'-(trifluoromethyl)-[4,5'-bipyrimidin]-2-yl)-4-methylpiperidin-4-ol
- 1-(2'-amino-6-(3-fluoroazetidin-1-yl)-4'-(trifluoromethyl)-[4,5'-bipyrimidin]-2-yl)-4-methylpiperidin-4-ol
- 1-(2'-amino-6-(3-methylazetidin-1-yl)-4'-(trifluoromethyl)-[4,5'-bipyrimidin]-2-yl)-4-methylpiperidin-4-ol
- 1-(2'-amino-6-(3,3-difluoroazetidin-1-yl)-4'-(trifluoromethyl)-[4,5'-bipyrimidin]-2-yl)-4-methylpiperidin-4-ol
- 1-(4-(6-amino-2-(trifluoromethyl)pyridin-3-yl)-6-(azetidin-1-yl)pyrimidin-2-yl)-4-methylpiperidin-4-ol
- 1-(4-(6-amino-2-(trifluoromethyl)pyridin-3-yl)-6-(3-fluoroazetidin-1-yl)pyrimidin-2-yl)-4-methylpiperidin-4-ol
- 1-(4-(6-amino-2-(trifluoromethyl)pyridin-3-yl)-6-(3-methylazetidin-1-yl)pyrimidin-2-yl)-4-methylpiperidin-4-ol
- 1-(4-(6-amino-2-(trifluoromethyl)pyridin-3-yl)-6-(3,3-difluoroazetidin-1-yl)pyrimidin-2-yl)-4-methylpiperidin-4-ol
- 1-(4-(6-amino-4-(trifluoromethyl)pyridin-3-yl)-6-(azetidin-1-yl)pyrimidin-2-yl)piperidin-4-one
- 1-(4-(6-amino-4-(trifluoromethyl)pyridin-3-yl)-6-(3-fluoroazetidin-1-yl)pyrimidin-2-yl)piperidin-4-one
- 1-(4-(6-amino-4-(trifluoromethyl)pyridin-3-yl)-6-(3-methylazetidin-1-yl)pyrimidin-2-yl)piperidin-4-one
- 1-(4-(6-amino-4-(trifluoromethyl)pyridin-3-yl)-6-(3,3-difluoroazetidin-1-yl)pyrimidin-2-yl)piperidin-4-one
- 1-(2'-amino-6-(azetidin-1-yl)-4'-(trifluoromethyl)-[4,5'-bipyrimidin]-2-yl)piperidin-4-one
- 1-(2'-amino-6-(3-fluoroazetidin-1-yl)-4'-(trifluoromethyl)-[4,5'-bipyrimidin]-2-yl)piperidin-4-one
- 1-(2'-amino-6-(3-methylazetidin-1-yl)-4'-(trifluoromethyl)-[4,5'-bipyrimidin]-2-yl)piperidin-4-one
- 1-(2'-amino-6-(3,3-difluoroazetidin-1-yl)-4'-(trifluoromethyl)-[4,5'-bipyrimidin]-2-yl)piperidin-4-one
- 1-(4-(6-amino-2-(trifluoromethyl)pyridin-3-yl)-6-(azetidin-1-yl)pyrimidin-2-yl)piperidin-4-one
- 1-(2'-amino-6-(3-fluoroazetidin-1-yl)-4'-(trifluoromethyl)-[4,5'-bipyrimidin]-2-yl)piperidin-4-one
- 1-(4-(6-amino-2-(trifluoromethyl)pyridin-3-yl)-6-(3-methylazetidin-1-yl)pyrimidin-2-yl)piperidin-4-one
- 1-(4-(6-amino-2-(trifluoromethyl)pyridin-3-yl)-6-(3,3-difluoroazetidin-1-yl)pyrimidin-2-yl)piperidin-4-one
- 5-(2-(4-aminopiperidin-1-yl)-6-(azetidin-1-yl)pyrimidin-4-yl)-4-(trifluoromethyl)pyridin-2-amine
- 5-(2-(4-aminopiperidin-1-yl)-6-(3-fluoroazetidin-1-yl)pyrimidin-4-yl)-4-(trifluoromethyl)pyridin-2-amine
- 5-(2-(4-aminopiperidin-1-yl)-6-(3-methylazetidin-1-yl)pyrimidin-4-yl)-4-(trifluoromethyl)pyridin-2-amine
- 5-(2-(4-aminopiperidin-1-yl)-6-(3,3-difluoroazetidin-1-yl)pyrimidin-4-yl)-4-(trifluoromethyl)pyridin-2-amine
- 2-(4-aminopiperidin-1-yl)-6-(azetidin-1-yl)-4'-(trifluoromethyl)-[4,5'-bipyrimidin]-2'-amine
- 2-(4-aminopiperidin-1-yl)-6-(3-fluoroazetidin-1-yl)-4'-(trifluoromethyl)-[4,5'-bipyrimidin]-2'-amine
- 2-(4-aminopiperidin-1-yl)-6-(3-methylazetidin-1-yl)-4'-(trifluoromethyl)-[4,5'-bipyrimidin]-2'-amine
- 2-(4-aminopiperidin-1-yl)-6-(3,3-difluoroazetidin-1-yl)-4'-(trifluoromethyl)-[4,5'-bipyrimidin]-2'-amine
- 5-(2-(4-aminopiperidin-1-yl)-6-(azetidin-1-yl)pyrimidin-4-yl)-6-(trifluoromethyl)pyridin-2-amine
- 5-(2-(4-aminopiperidin-1-yl)-6-(3-fluoroazetidin-1-yl)pyrimidin-4-yl)-6-(trifluoromethyl)pyridin-2-amine
- 5-(2-(4-aminopiperidin-1-yl)-6-(3-methylazetidin-1-yl)pyrimidin-4-yl)-6-(trifluoromethyl)pyridin-2-amine
- 5-(2-(4-aminopiperidin-1-yl)-6-(3,3-difluoroazetidin-1-yl)pyrimidin-4-yl)-6-(trifluoromethyl)pyridin-2-amine

13. The compound of formula (I) according to claim 1, selected from the group consisting of
- 5-(6-(azetidin-1-yl)-2-morpholinopyrimidin-4-yl)-4-(trifluoromethyl)pyridin-2-amine
- 5-(6-(3-fluoroazetidin-1-yl)-2-morpholinopyrimidin-4-yl)-4-(trifluoromethyl)pyridin-2-amine
- 5-(6-(3-methylazetidin-1-yl)-2-morpholinopyrimidin-4-yl)-4-(trifluoromethyl)pyridin-2-amine
- 5-(6-(3,3-difluoroazetidin-1-yl)-2-morpholinopyrimidin-4-yl)-4-(trifluoromethyl)pyridin-2-amine
- 6-(3,3-difluoroazetidin-1-yl)-2-morpholino-4'-(trifluoromethyl)-[4,5'-bipyrimidin]-2'-amine
- (*R*)-5-(6-(3,3-difluoroazetidin-1-yl)-2-(3-methylmorpholino)pyrimidin-4-yl)-4-(trifluoromethyl)pyridin-2-amine
- 1-(4-(6-amino-4-(trifluoromethyl)pyridin-3-yl)-6-(3,3-difluoroazetidin-1-yl)pyrimidin-2-yl)piperidin-4-ol
- 1-(4-(6-amino-4-(trifluoromethyl)pyridin-3-yl)-6-(3,3-difluoroazetidin-1-yl)pyrimidin-2-yl)piperidin-4-one;
and tautomers, solvates and pharmaceutically acceptable salts thereof.

14. A pharmaceutical composition comprising a compound of formula (I) according to any one of claims 1 to 13 and a pharmaceutically acceptable carrier.

15. A method of preventing or treating a hyperproliferative disease or disorder, comprising administering to a mammal in need of such treatment an effective amount of a compound of formula (I) as claimed in any one of the claims 1 to 13, alone or in combination with one or more additional compounds having anti-hyperproliferative properties.
